# EUROPEAN PATENT APPLICATION

(11) **EP 4 349 963 A1**
(43) Date of publication of application: **10.04.2024**
(21) Application number: 22816495.0
(22) Date of filing: 03.06.2022
(51) Int. Cl.: C12N 5/00, C12N 5/0775, C12N 5/0797, C12N 5/0789, A61K 35/28, A61P 29/00, A61P 37/02, A61P 17/02

(54) **METHOD FOR ISOLATING AND CULTURING TISSUE-RESIDENT UPAR+/NESTIN+ STEM CELLS, AND USE THEREOF**

(30) Priority: 03.06.2021 KR 20210072137; 02.06.2022 KR 20220067722
(71) Applicant: Innostem Bio, Busan 47392 (KR)
(72) Inventor: KWAK, Myeong Jin, Busan 46276 (KR)
(74) Representative: Roos, Peter
(86) International application number: PCT/KR2022/007910
(87) International publication number: WO 2022/255836

(57) **Abstract**

The present invention relates to a method for isolating and culturing tissue-resident uPAR+/Nestin+ stem cells, and a use thereof, and provides a method for isolating, on the basis of uPAR-plasmin activity, tissue-resident stem cells present in solid tissues. The uPAR-plasmin activity of stem cells has been suggested to have a close relationship with stem cell growth, migration ability, physiological activity and differentiation ability, and thus can be used in a method for isolating high-potency stem cells. The uPAR+ stem cells, of the present invention, isolated from solid tissues, are applicable to the production of cell therapeutic agents, tissue engineering therapeutic agents, and novel biopharmaceuticals using secretions including exosomes, and thus have high industrial applicability. In addition, the present invention is applicable to fundamental cell biology and molecular biology research and novel drug development research that relate to the cell division, migration, growth, and differentiation of tissue-resident stem cells.

## Description

### Technical Field

The present disclosure relates to a method of isolating and culturing tissue-resident uPAR+/Nestin+ stem cells that play an integral role in tissue regeneration and tissue homeostasis in solid tissues, and a use thereof.

### Background Art

A conventional method for isolating and culturing stem cells present in solid tissues requires a process of preparing a single cell suspension after undergoing tissue dissociation to degrade tissues through protease treatment and separating each cell constituting the tissue. In the tissue dissociation process, a single cell yield obtained based on the type of proteases used, concentration, reaction time, reaction temperature, and type of tissues used shows a large variation, leaving an unavoidable problem concerning cell damage during the tissue dissociation process. As a result, the tissue dissociation process shows a significant difference in a cell yield and the degree of cell injury depending on the tissue type, with a disadvantage that is difficult to standardize. In particular, with less than 0.1% frequency of stem cells in solid tissues, it is widely known that tissue dissociation steps following a protease treatment result in in extremely low cell yield.

A follow-up process of isolating and purifying tissue-resident stem cells from the single cell suspension dissociated from tissues is required. The process of isolating and purifying stem cells involves the use of markers such as c-Kit and Sca-1 to isolate cells whose markers are positive or negative. However, since these markers are expressed not only in tissue-resident stem cells but also in hematopoietic cells, unwanted cells may be separated altogether, and there are stem cells whose markers are negative, thereby having a limit on the method of isolating and purifying tissue-resident stem cells using specific markers.

### Disclosure of the Invention

### Technical Goals

An object of the present disclosure is to provide a method of inducing cells to enter cell cycle of tissue-resident uPAR+/nestin+ stem cells located in solid tissues such as adipose tissue, bone marrow tissue, myocardial tissue, peripheral nerve tissue, skeletal muscle tissue, or synovial tissue, inducing migration and growth, and isolating cells.

In addition, another object of the present disclosure is to provide tissue-resident uPAR+ and nestin+ stem cells isolated and cultured according to the method, or a culture thereof.

In addition, another object of the present disclosure is to provide a pharmaceutical composition for preventing or treating inflammatory diseases or autoimmune diseases, including the tissue-resident uPAR+ and nestin+ stem cells or a culture thereof as an active ingredient.

In addition, another object of the present disclosure is to provide a pharmaceutical composition for healing wounds or promoting vascular regeneration, including the tissue-resident uPAR+ and nestin+ stem cells or a culture thereof as an active ingredient.

### Technical Solutions

In order to achieve the above objects, the present disclosure provides a method of inducing tissue-resident uPAR+ and nestin+ stem cells to enter the cell cycle including: (1) preparing a provisional matrix-mimicking hydrogel; (2) encapsulating isolated tissue fragments into the provisional matrix-mimicking hydrogel; and (3) three-dimensional (3D) culturing the provisional matrix-mimicking hydrogel into which the tissue fragments are encapsulated, in a culture medium to which plasminogen activator inhibitor (PAI) is added.

In addition, the present disclosure provides a method of isolating and culturing tissue-resident uPAR+ and nestin+ stem cells including: (1) preparing a provisional matrix-mimicking hydrogel; (2) encapsulating isolated tissue fragments into the provisional matrix-mimicking hydrogel; (3) 3D culturing the provisional matrix-mimicking hydrogel into which the tissue fragments are encapsulated, in a culture medium to which PAI is added; (4) removing the 3D culture medium and removing PAI by washing; (5) re-culturing the PAI-removed culture with a PAI-free culture medium to degrade the provisional matrix-mimicking hydrogel; and (6) isolating stem cells released in the re-culture medium.

In addition, the present disclosure provides tissue-resident uPAR+ and nestin+ stem cells isolated and cultured according to the method, or a culture thereof.

In addition, the present disclosure provides a pharmaceutical composition for preventing or treating inflammatory diseases, including the tissue-resident uPAR+ and nestin+ stem cells or a culture thereof as an active ingredient.

In addition, the present disclosure provides a pharmaceutical composition for preventing or treating autoimmune diseases, including the tissue-resident uPAR+ and nestin+ stem cells or a culture thereof as an active ingredient.

In addition, the present disclosure provides a pharmaceutical composition for healing wounds, including the tissue-resident uPAR+ and nestin+ stem cells or a culture thereof as an active ingredient.

In addition, the present disclosure provides a pharmaceutical composition for promoting vascular regeneration, including the tissue-resident uPAR+ and nestin+ stem cells or a culture thereof as an active ingredient.

### Advantageous Effects

The present disclosure relates to a method of isolating and culturing tissue-resident uPAR+/Nestin+ stem cells and a use thereof, providing a method of isolating based on a uPAR-plasmin activity of tissue-resident stem cells present in solid tissues. The uPAR-plasmin activity of stem cells is closely related to a growth, migratory ability, biochemical activity, and differentiation ability of stem cells and thus is applicable as a method of isolating stem cells with the high level of functional activity. uPAR+ stem cells isolated from solid tissues of the present disclosure have great industrial potential owing to applicability to production of cell therapeutic agents, tissue engineering therapeutic agents, and new biopharmaceuticals using secretions including exosomes. In addition, in the present disclosure, it may be applied to basic research on cell biology and molecular biology related to cell division, migration, growth, and differentiation of tissue-resident stem cells and new drug development research.

### Brief Description of Drawings

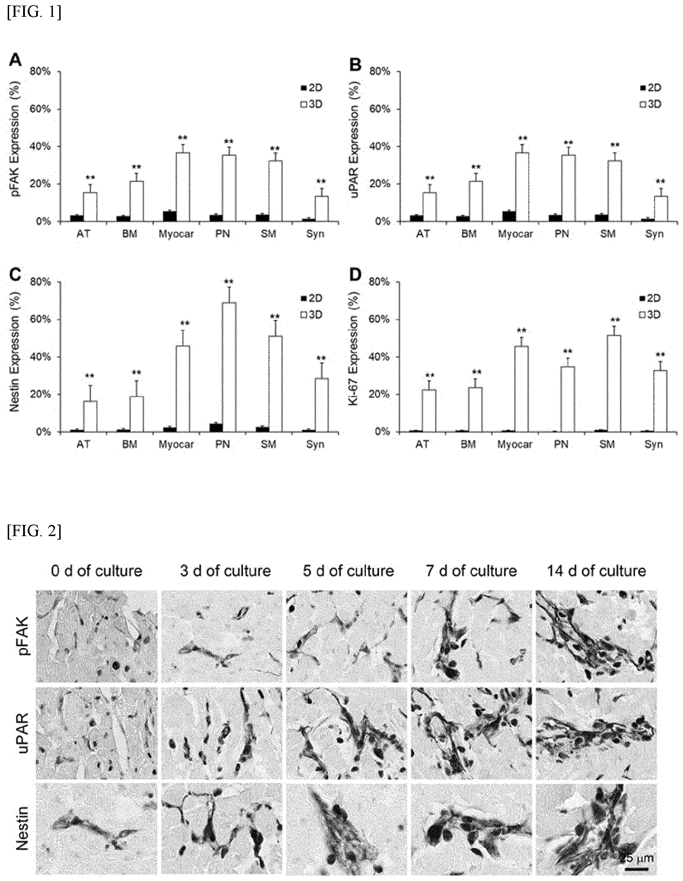

FIG. 1 shows expression rates of pFAK, uPAR, nestin, and Ki-67 of constituent cells in tissue fragments in vitro organ culture. 2D, monolayer organ culture; 3D, 3D organ culture; AT, adipose tissue; BM, bone marrow; Myocar, myocardium; PN, peripheral nerve; SM, skeletal muscle; Syn, Synovium. **, p < 0.01 vs. 2D.
FIG. 2 shows characteristics of which pFAK+, uPAR+, and Nestin+ expressing cells in myocardium increase according to 3D myocardial organ culture.

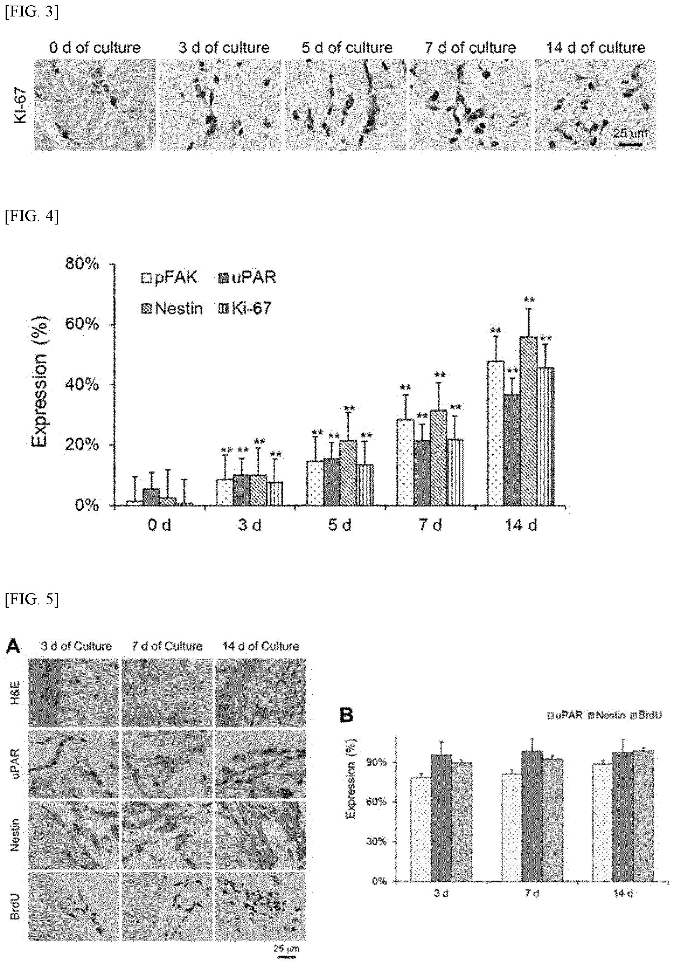

FIG. 3 shows characteristics of which ki-67 positive cells having cell division in myocardium increase according to 3D organ culture.
FIG. 4 shows that the frequency of pFAK, uPAR, nestin, and ki-67 positive cells in myocardium increase in proportion to a period of 3D myocardial organ culture. **, p < 0.01 vs. 0 d (before culture).
FIG. 5 shows expression of uPAR, nestin, and BrdU of cells migrated into and grown in a hydrogel after 3D myocardial organ culture.

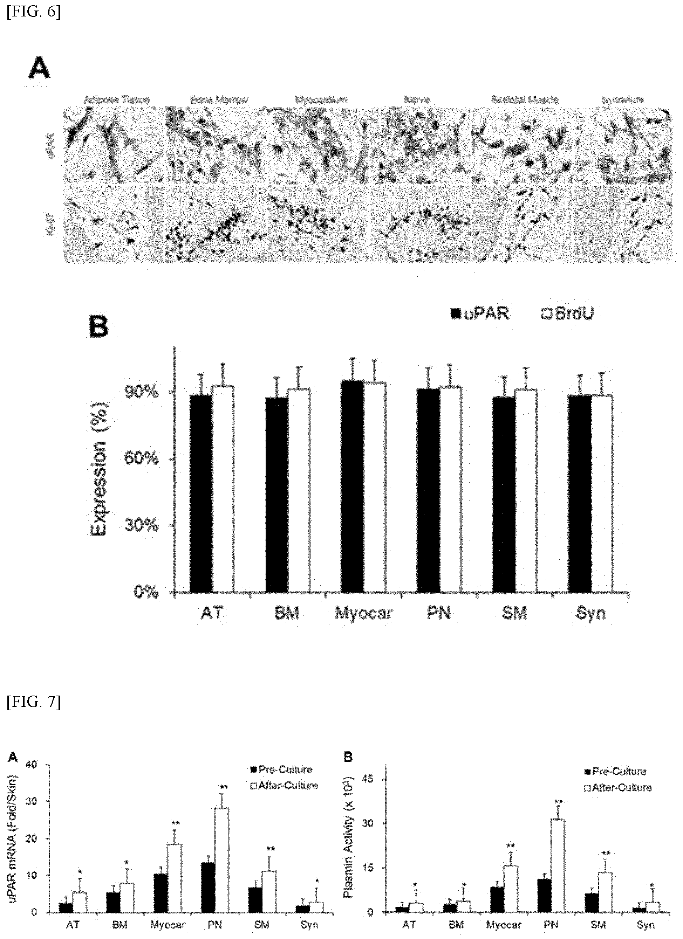

FIG. 6 shows expression rates of uPAR and nestin of cells migrated into and grown in a hydrogel after 2 weeks of 3D organ culture. AT, adipose tissue; BM, bone marrow; Myocar, myocardium; PN, peripheral nerve; SM, skeletal muscle; Syn, synovium.
FIG. 7 shows uPAR mRNA expression (A) and plasmin activity (B) in tissue fragments before organ culture and 3 days after organ culture. AT, adipose tissue; BM, bone marrow; Myocar, myocardium; PN, peripheral nerve; SM, skeletal muscle; Syn, synovium. *, p < 0.05 vs. pre-culture (before culture); **, p < 0.01 vs. pre-culture (before culture).

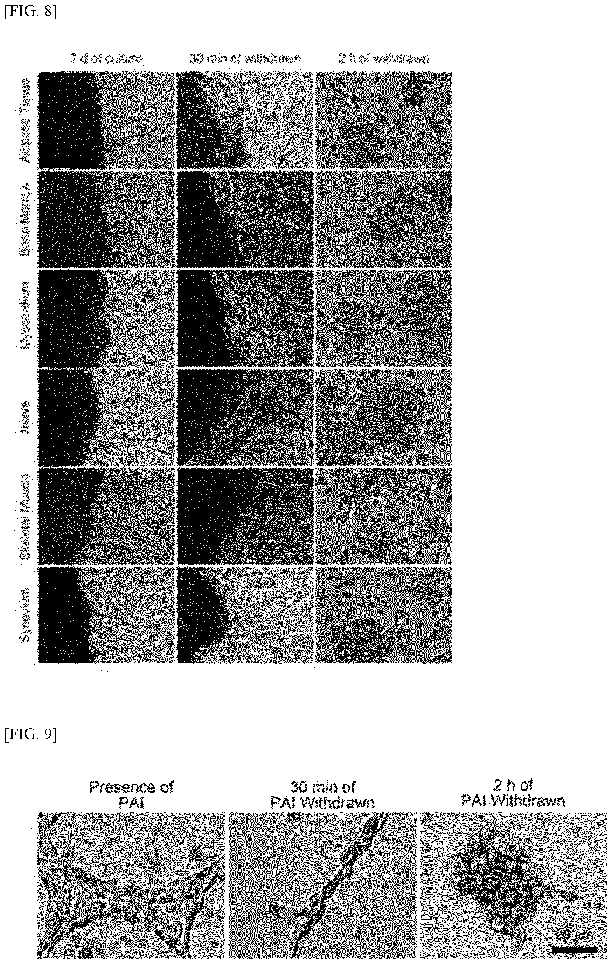

FIG. 8 shows that hydrogel is degraded through PAI removal and culture washing in a culture medium after organ culture, and cells migrated into and grown in the hydrogel are released into the culture medium.
FIG. 9 shows that cells migrated into and grown in a hydrogel are detached from hydrogel through PAI removal and culture washing in a culture medium, and the released cells shrink and aggregate (phase contrast micrograph).

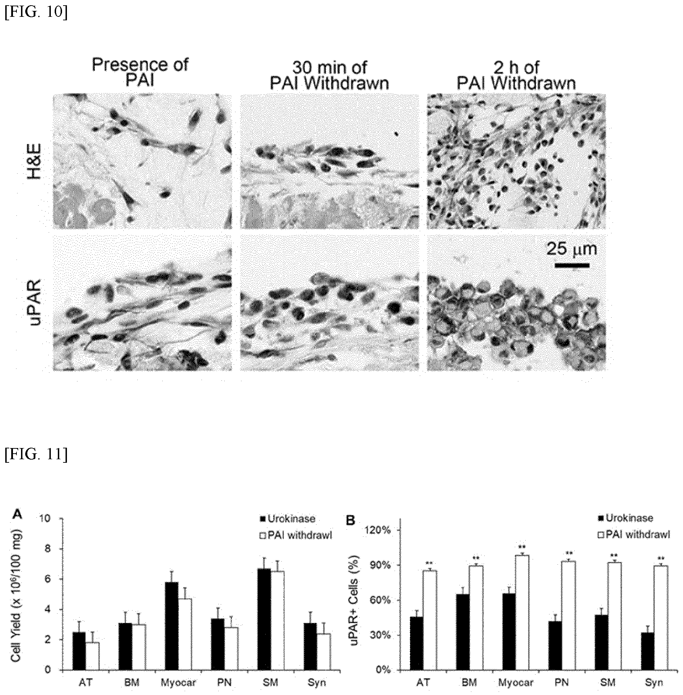

FIG. 10 shows separation in that uPAR+ cells in a hydrogel are released after PAI removal and culture washing (HE staining of paraffin sections and uPAR immunohistochemistry staining).
FIG. 11 shows yields of cells collected after isolation from hydrogel after PAI removal, culture washing (PAI withdrawn), and exogenous urokinase addition (Urokinase) and an expression rate of uPAR in collected cells. AT, adipose tissue; BM, bone marrow; Myocar, myocardium; PN, peripheral nerve; SM, skeletal muscle; Syn, synovium. **, p < 0.01 vs. Urokinase.

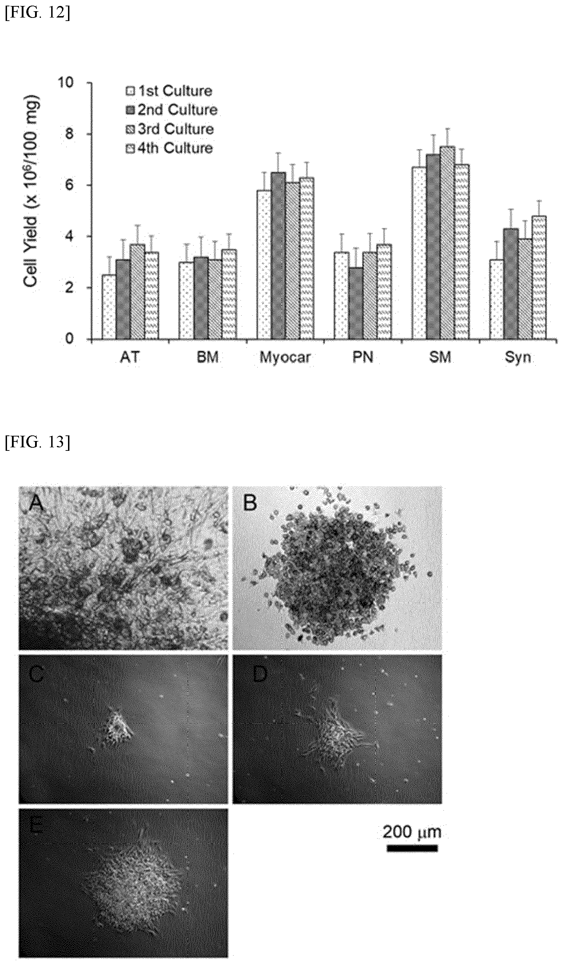

FIG. 12 shows yields of cells collected after repeated isolation from hydrogel through repeated organ culture of tissue fragments collected after PAI removal and culture washing. AT, adipose tissue; BM, bone marrow; Myocar, myocardium; PN, peripheral nerve; SM, skeletal muscle; Syn, synovium.
FIG. 13 shows adhesion and growth of cells in a monolayer culture environment after seeding cells collected after PAI removal and culture washing in a culture vessel. A, Aggregates of cells released from hydrogel after PAI removal and washing. B, Aggregates of cells collected from hydrogels. C-E, Adhesion and growth of cells at 30 minutes (C), 1 hour (D), and 2 hours (D) after seeding collected cells in a culture vessel (phase contrast micrograph).

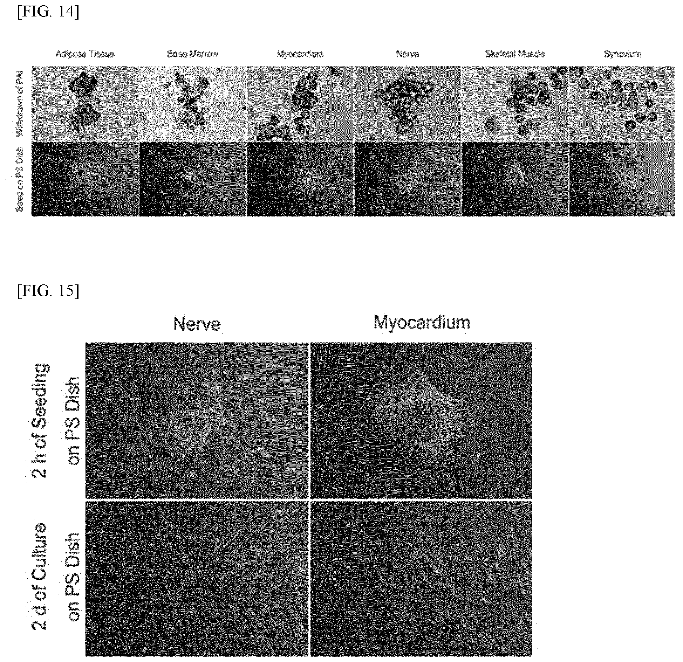

FIG. 14 shows cells released from hydrogel after culture of adipose tissues, bone marrow, myocardium, nerves, skeletal muscle, and synovial organs, followed by PAI removal and washing of the culture (top) and results of collected cells being seeded, attached to a culture vessel, and grown (bottom) (phase contrast micrograph).
FIG. 15 shows adhesion and growth in a monolayer culture environment of cells released from hydrogel after PAI removal and culture washing after nerve and myocardial organ culture.

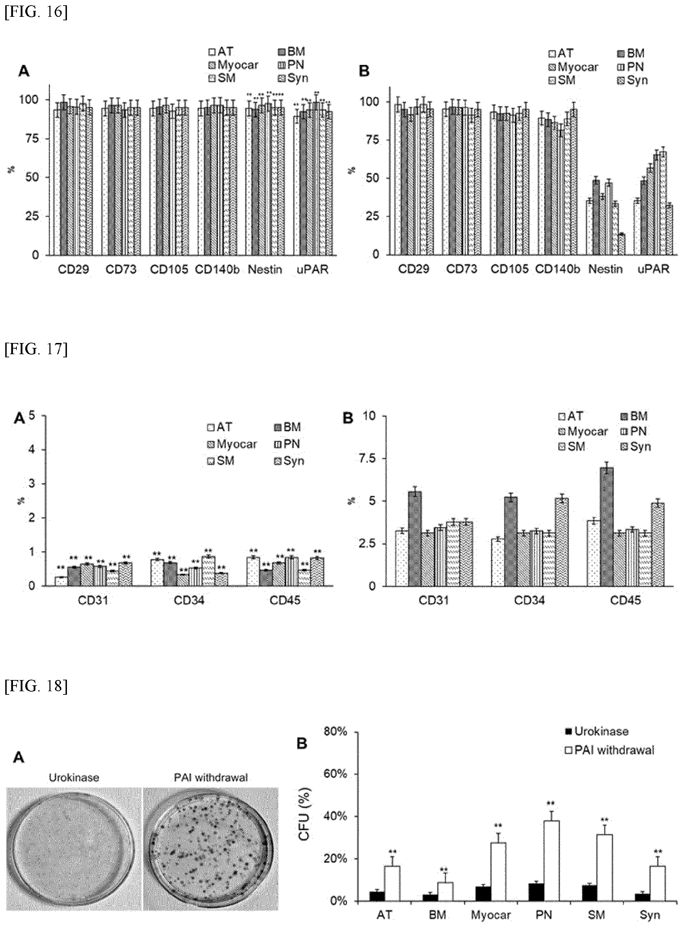

FIG. 16 shows immunophenotypic features of cells collected from hydrogel. A, Immunophenotypic features of cells released after withdrawal from hydrogel after PAI removal and culture washing. B, Immunophenotypic features of cells released from hydrogel after exogenous urokinase treatment. AT, adipose tissue; BM, bone marrow; Myocar, myocardium; PN, peripheral nerve; SM, skeletal muscle; Syn, synovium. **, p < 0.01 vs. urokinase (B).
FIG. 17 shows expression rates of hematopoietic cell and vascular endothelial cell markers of cells collected from hydrogel. A, Expression rate of cells isolated and collected after PAI removal and culture washing. B, Expression rate of cells isolated and collected after exogenous urokinase treatment. AT, adipose tissue; BM, bone marrow; Myocar, myocardium; PN, peripheral nerve; SM, skeletal muscle; Syn, synovium. **, p < 0.01 vs. urokinase (B).
FIG. 18 shows a self-replication ability of cells isolated and collected from hydrogel. A; Representative picture of colony forming unit (CFU). B; CFU frequency of cells isolated and collected from hydrogel according to tissue origin. Urokinase, cells isolated and collected after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. **, p < 0.01 vs. urokinase.

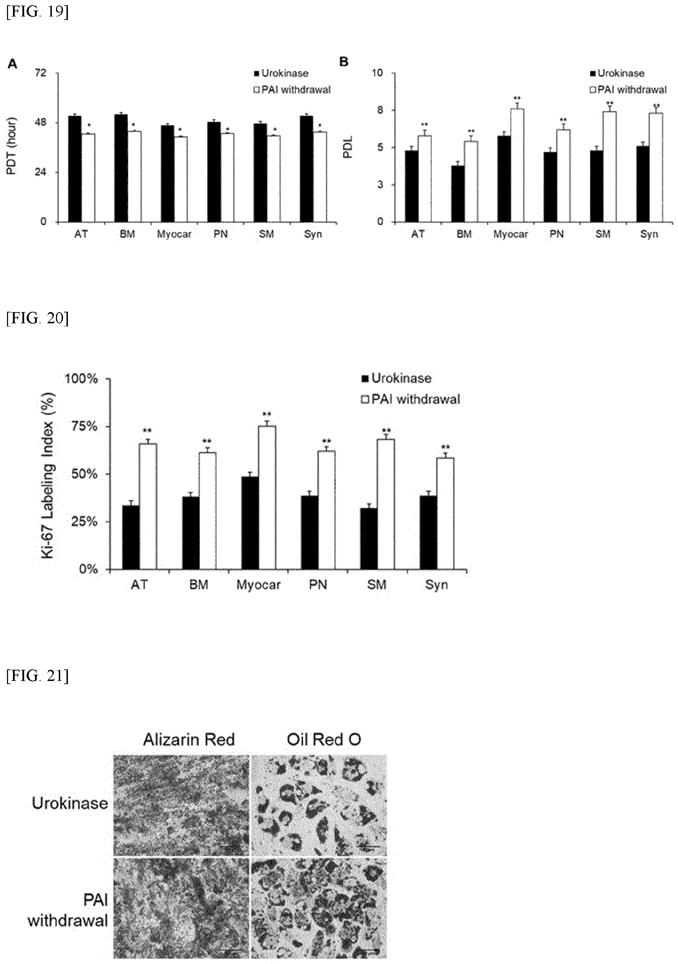

FIG. 19 shows an in vitro growth ability of cells isolated and collected from hydrogel. A; Population Doubling Time (PDT). B; Population Doubling Level (PDL). Urokinase, cells isolated and collected after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. urokinase; **, p < 0.01 vs. urokinase.
FIG. 20 shows a Ki-67 expression rate of cells isolated and collected from hydrogel. Urokinase, cells isolated and collected after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. **, p < 0.01 vs. urokinase.
FIG. 21 shows an ability of cells isolated and collected from hydrogel to differentiate into osteoblasts (alizarin red) and adipocytes (oil red O). Urokinase, cells isolated and collected after exogenous urokinase treatment; PAI withdrawal, cells isolated and collected after PAI removal and culture washing.

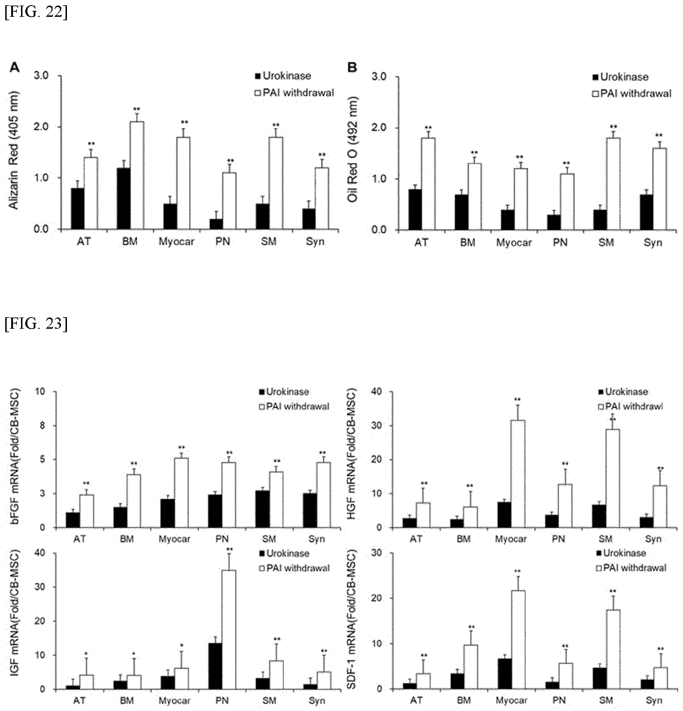

FIG. 22 shows comparisons of an ability of cells isolated and collected from hydrogel to differentiate into osteoblasts (alizarin red) and adipocytes (oil red O). Urokinase, cells isolated and collected after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. **, p < 0.01 vs. urokinase.
FIG. 23 shows the expression levels of mRNAs related with tissue regeneration, stem cell mobilization, and angiogenesis of cells isolated and collected from hydrogel. Urokinase, cells isolated and collected after exogenous urokinase treatment; PAI withdrawal, cells isolated and collected after PAI removal and culture washing; CB-MSC, cord blood-derived mesenchymal stem cell; AT, adipose tissue; BM, bone marrow; Myocar, myocardium; PN, peripheral nerve; SM, skeletal muscle; Syn, synovium. *, p < 0.05 vs. urokinase; **, p < 0.01 vs. urokinase.

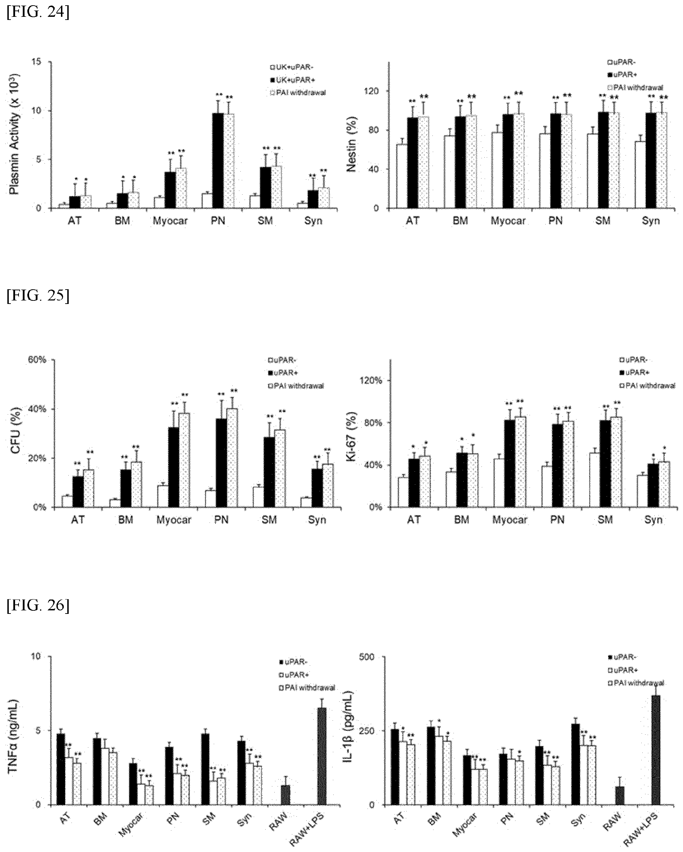

FIG. 24 shows a plasmin activity and nestin expression rate of cells isolated and collected from hydrogel. uPAR-, uPAR negative cells isolated and purified after exogenous urokinase treatment; uPAR+, uPAR+ cells isolated and purified after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. uPAR-; **, p , 0.01 vs. uPAR-. *, p < 0.05 vs. uPAR-; **, p < 0.01 vs. uPAR-.
FIG. 25 shows a self-replication (colony forming unit; CFU) and in vitro growth (Ki-67) ability of cells isolated and collected from hydrogel. uPAR-, uPAR negative cells isolated and purified after exogenous urokinase treatment; uPAR+, uPAR+ cells isolated and purified after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. uPAR-; **, p , 0.01 vs. uPAR-. *, p < 0.05 vs. uPAR-; **, p < 0.01 vs. uPAR-.
FIG. 26 shows an anti-inflammatory ability of cells isolated and collected from hydrogel. TNFα and IL-1β secretion inhibitory effect of RAW 264.7 cells sensitized by LPS in a conditioned medium. uPAR-, uPAR negative cells isolated and purified after exogenous urokinase treatment; uPAR+, uPAR+ cells isolated and purified after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. uPAR-; **, p , 0.01 vs. uPAR-. *, p < 0.05 vs. uPAR-; **, p < 0.01 vs. uPAR-.

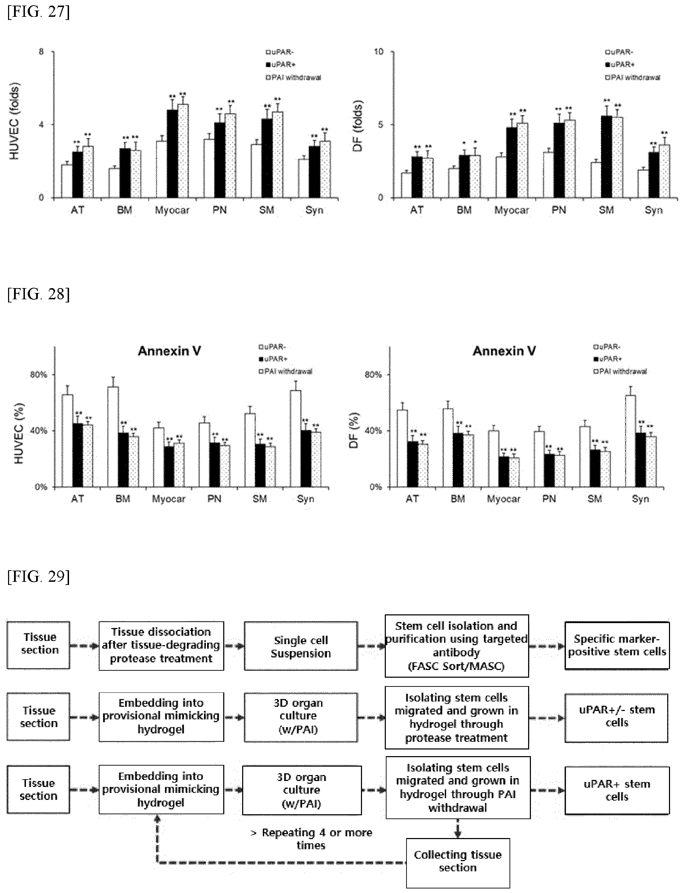

FIG. 27 shows a vascular endothelial cell (HUVEC) and fibroblast (DF) growth stimulation activity of cells isolated and collected from hydrogel. uPAR-, uPAR negative cells isolated and purified after exogenous urokinase treatment; uPAR+, uPAR+ cells isolated and purified after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. uPAR-; **, p , 0.01 vs. uPAR-. *, p < 0.05 vs. uPAR-; **, p < 0.01 vs. uPAR-.
FIG. 28 shows a vascular endothelial cell (HUVEC) and fibroblast (DF) cytoprotective activity of cells isolated and collected from hydrogel. uPAR-, uPAR negative cells isolated and purified after exogenous urokinase treatment; uPAR+, uPAR+ cells isolated and purified after exogenous urokinase treatment; PAI withdrawal, cells isolated and collected after PAI removal and culture washing; AT, adipose tissue; BM, bone marrow; Myocar, myocardium; PN, peripheral nerve; SM, skeletal muscle; Syn, synovium. *, p < 0.05 vs. uPAR-; **, p , 0.01 vs. uPAR-. *, p < 0.05 vs. uPAR-; **, p < 0.01 vs. uPAR-.
FIG. 29 shows a schematic diagram of the present disclosure compared to the prior art.

### Best Mode for Carrying Out the Invention

The present disclosure provides a method of inducing tissue-resident uPAR+ and nestin+ stem cells to enter the cell cycle including: (1) preparing a provisional matrix-mimicking hydrogel; (2) encapsulating isolated tissue fragments into the provisional matrix-mimicking hydrogel; and (3) 3D culturing the provisional matrix-mimicking hydrogel into which the tissue fragments are encapsulated, in a culture medium to which plasminogen activator inhibitor (PAI) is added.

Preferably, the provisional matrix-mimicking hydrogel may be a fibrin hydrogel in which a fibrinogen solution at a concentration of 0.25 to 2.5% and a thrombin solution at a concentration of 0.5 to 5 I.U./mL are mixed, a fibrin/collagen mixed hydrogel in which a collagen solution at a concentration of 0.1 to 0.5% is mixed in the fibrin hydrogel, or a fibrin/gelatin mixed hydrogel in which a gelatin solution at a concentration of 0.1 to 0.5% is mixed in the fibrin hydrogel, but is not limited thereto.

Preferably, the tissue may be adipose tissue, bone marrow tissue, myocardial tissue, peripheral nerve tissue, skeletal muscle tissue, and synovial tissue, but is not limited thereto.

Preferably, the PAI may be tranexamic acid or aminomethyl benzoic acid, but is not limited thereto.

Preferably, the method may activate integrin-FAK cell signaling of cells in tissues to induce cell division and cell growth of tissue-resident uPAR+ and nestin+ stem cells, but is not limited thereto.

Preferably, the method may induce cell migration into and cell growth in the provisional matrix-mimicking hydrogel of the tissue-resident uPAR+ and nestin+ stem cells, but is not limited thereto.

A tissue-specific provisional matrix is required to induce migration of uPAR+ and nestin+ tissue-resident stem cells of the present disclosure into the provisional matrix. By mimicking provisional matrix components which are formed as plasma released from blood vessels after tissue damage is coagulated, preparation may be performed using biopolymers. The provisional matrix of the present disclosure may be prepared using fibrin, collagen, and gelatin alone or in combination thereof. The uPA-plasmin activity of the engineered provisional matrix varies depending on the organ, and the provisional matrix resistant to uPA-plasmin activity may be prepared to be used. The provisional matrix may be prepared by adjusting a content or composition of biopolymers. In other words, provisional matrix obtained by mixing two or more components, such as fibrin-collagen, fibrin-gelatin, and collagen-gelatin, may be used. The provisional matrix of the present disclosure may contain constituent polymers such as fibrinogen, collagen, and gelatin in an amount of 1.0 to 20.0 mg/ml. In order to strengthen the structural properties of the provisional matrix, the crosslinking degree may be adjusted, and the structural properties may be adjusted by controlling a degree of crosslinking of the provisional matrix using a crosslinking agent such as Ca++ or factor XIIIa.

Activation of cell signaling pathways is required to induce cell division and growth of stem cells in tissues through in vitro culture. In combination with the integrin ligand of the cell, a signal that is capable of inducing cell growth and migration may be transmitted and activated through the provisional matrix. By supporting tissues with the provisional matrix capable of transmitting signals into cells by directly binding to integrins of cells, it is possible to induce cell division, growth, and migration of stem cells in tissues. The integrin-β1-FAK signaling pathway transmits signals to tissue-resident stem cells through integrin and induces activation owing to the support by the provisional matrix, and tissue-resident stem cells may increase uPAR and nestin expression according to the transmitted signal and induce division, growth, and migration of regenerative stem cells in tissues. The provisional matrix of the present disclosure capable of activating the integrin-FAK cell signaling pathway may achieve the above object by preparing the provisional matrix-mimicking hydrogel composed of polymers having RGD motifs such as fibrin, collagen, and gelatin.

The present disclosure provides a method of inducing cell division, growth, and migration of tissue-resident stem cells by activating the integrin-β1-FAK-uPAR signaling pathway. The efficiency of signaling to stem cells in tissues is proportional to a density of receptors capable of binding to integrin ligands. The density of receptors capable of binding to integrin ligand may be achieved by increasing a contact surface with the tissue. By providing a three-dimensional (3D) environment rather than two-dimensional (2D) to increase an area to adhere to the cell consequently, binding of integrin ligands and receptors may be increased. As a result, it is possible to increase the signaling efficiency to stem cells in tissues. The present disclosure provides a method of activating the integrin-β1-FAK-uPAR signaling pathway by providing a 3D support of an artificial provisional matrix to tissues. To this end, provided is a method of strengthening a signaling pathway by providing 3D bond to tissue fragments with hydrogel capable of sol-gel phase transition as provisional matrix.

uPA-plasmin activity may vary depending on the tissue, the degree of damage and the cause. Compared with fat, placenta, and umbilical cord, nervous tissues have high uPA-plasmin activity, and as a result, the nervous tissues have a high degree of degradation for provisional matrix. The degradation of the provisional matrix may be controlled by adjusting the content of the components constituting the provisional matrix, a molecular weight of a polymer, and the degree of crosslinking density. The present disclosure provides a method of controlling a degree of resistance to uPA-plasmin activity to be raised by increasing a concentration, molecular weight, and degree of crosslinking of a polymer constituting the provisional matrix. In addition, the present disclosure provides a method of regulating uPA-plasmin activity through PAI addition to continuously induce the activity, growth, and migration of stem cells in tissues in the provisional matrix. PAI applicable to the present disclosure may be selected from the group consisting of aminocaproid acid, tranexamic acid, aprotinin, and aminomethylbenzoic acid. The PAI applied to the present disclosure may be used by addition at a concentration of 10 µg ~ 10 mg per ml of a hydrogel dose. As a result, the present disclosure provides a method of inducing and supporting growth and migration of tissue-resident stem cells during organ culture by maintaining addition of PAI and a structural stability of the provisional matrix.

For an excessive activity of uPAR-uPA-plasmin of stem cells, as a result of rapid degradation of provisional matrix-mimicking hydrogel during the in vitro culture process, tissue fragments and pericellular provisional matrix are degraded and lost to cause a loss of the provisional matrix to which stem cells may attach and migrate, resulting in the loss, reduction, or loss of cell migration from the tissue into the hydrogel. In order to control excessive degradation and loss of the provisional matrix-mimicking hydrogel, the present disclosure provides a method of maintaining and sustaining a structural functioning as a matrix on which the provisional matrix mimicking-hydrogel maintains a role as a physical support matrix for tissue fragments during organ culture period, while activated tissue-resident stem cells are able to migrate and grow, by controlling the excessive plasmin activity of stem cells and tissue fragments by adding PAI.

In addition, the present disclosure provides a method of isolating and culturing tissue-resident uPAR+ and nestin+ stem cells including: (1) preparing a provisional matrix-mimicking hydrogel; (2) encapsulating isolated tissue fragments into the provisional matrix-mimicking hydrogel; (3) 3D culturing the provisional matrix-mimicking hydrogel into which the tissue fragments are encapsulated, in a culture medium to which PAI is added; (4) removing the 3D culture medium and removing PAI by washing; (5) re-culturing the PAI-removed culture with a PAI-free culture medium to degrade the provisional matrix-mimicking hydrogel; and (6) isolating stem cells released in the re-culture medium.

Preferably, the provisional matrix-mimicking hydrogel is a fibrin hydrogel in which a fibrinogen solution at a concentration of 0.25 to 2.5% and a thrombin solution at a concentration of 0.5 to 5 I.U./mL are mixed, a fibrin/collagen mixed hydrogel in which a collagen solution at a concentration of 0.1 to 0.5% is mixed in the fibrin hydrogel, or a fibrin/gelatin mixed hydrogel in which a gelatin solution at a concentration of 0.1 to 0.5% is mixed in the fibrin hydrogel, but is not limited thereto.

Preferably, the tissue may be adipose tissue, bone marrow tissue, myocardial tissue, peripheral nerve tissue, skeletal muscle tissue, or synovial tissue, but is not limited thereto.

Preferably, the PAI may be tranexamic acid or aminomethyl benzoic acid, but is not limited thereto.

Preferably, step (5) may induce an increase in uPAR expression in the tissue and degrade the provisional matrix-mimicking hydrogel through an increase in plasmin activity, but is not limited thereto.

Preferably, the tissue-resident uPAR+ and nestin+ stem cells may have increased ability in self-replication, in vitro growth, differentiation, or tissue regeneration induction, but are not limited thereto.

Preferably, the tissue fragments collected from the re-culture medium in step (5) may further include a process of repeating steps (2) to (5) 1 to 10 times, but the present disclosure is not limited thereto.

The present disclosure provides a method of selectively isolating uPAR+ stem cells migrated into and grown in the provisional matrix. Though it is possible to control degradation of the provisional matrix by excessive uPAR-plasmin activity through PAI addition, provided is a method of collecting cells from provisional matrix after migration and growth of target stem cells are achieved. Using the properties of stem cell intrinsic uPAR-plasmin activity by removing PAI after washing, provided is a method of collecting released stem cells migrated into and grown in the matrix as the provisional matrix is degraded. The present disclosure provides a method of isolating uPAR+ stem cells from solid tissue without using any exogenous protease and without subsequent purification process, with degradation of provisional matrix according to a uPAR expression rate and plasmin activity in stem cells by uPAR-plasmin.

The present disclosure provides a method of collecting in a state in which a structure and function of tissue fragments used in organ culture are kept intact by not using any exogenous protease. Provided is a method of collecting tissue-resident uPAR+ stem cells by inducing migration into provisional matrix through repeated organ culture as structures of tissue fragments collected after organ culture are preserved.

The present disclosure provides a composition of a provisional matrix in which a uPAR-uPA-plasmin activity of tissue-resident stem cells varies depending on the tissue without degradation and loss due to the uPAR-uPA-plasmin activity according to the tissue, and also provides a type and concentration of PAI according to the tissue in which excessive degradation is controllable.

Provided is a method of isolating high-purity stem cells without subsequent purification process by performing PAI rinsing and removal (PAI withdrawal) of tissue-resident uPAR+/nestin+ stem cells migrated into and grown in the provisional matrix-mimicking hydrogel. The tissue-resident uPAR+/nestin+ stem cells may be used in production of stem cell therapeutic agents and stem cell-derived biopharmaceuticals with high effects of self-replication, in vitro growth, differentiation ability, tissue regeneration-inducing gene expression, vascular regeneration, and wound healing.

In addition, the present disclosure provides tissue-resident uPAR+ and nestin+ stem cells isolated and cultured according to the method, or a culture thereof.

In the present disclosure, the term "culture medium" as used herein refers to a medium capable of supporting stem cell growth and survival in vitro and includes secretions of cultured stem cells included in the medium. The medium used for culture includes all conventional media used in the art, suitable for stem cell culture. Depending on the type of cells, the medium and culture conditions may be selected. The medium used for culture is preferably a cell culture minimum medium (CCMM), generally including a carbon source, a nitrogen source, and a trace element component. Such cell culture minimum media include, but are not limited to, for example, Dulbecco's Modified Eagle's Medium (DMEM), Minimal Essential Medium (MEM), Basal Medium Eagle (BME), RPMI1640, F-10, F-12, α Minimal Essential Medium (αMEM), Glasgow's Minimal essential Medium (GMEM), and Iscove's Modified Dulbecco's Medium.

On the other hand, in the present disclosure, usable forms include a form all including the stem cells, secretions thereof, and medium components, a form including only secretions and medium components, a form using isolated secretions alone or in combination with stem cells, or a form producing secretions in vivo by administering only stem cells.

The stem cells may be obtained using any method commonly known in the art.

In addition, the present disclosure provides a pharmaceutical composition for preventing or treating inflammatory diseases including the tissue-resident uPAR+ and nestin+ stem cells or a culture thereof as an active ingredient.

In addition, the present disclosure provides a pharmaceutical composition for preventing or treating autoimmune diseases including the tissue-intrinsic uPAR+ and nestin+ stem cells or a culture thereof as an active ingredient.

In addition, the present disclosure provides a pharmaceutical composition for healing wounds including the tissue-resident uPAR+ and nestin+ stem cells or a culture thereof as an active ingredient.

In addition, the present disclosure provides a pharmaceutical composition for promoting vascular regeneration including the tissue-resident uPAR+ and nestin+ stem cells or a culture thereof as an active ingredient.

The tissue-resident uPAR+ and nestin+ stem cells of the present disclosure may be used as cell therapeutic agents for treatment of certain diseases, the treatment of which may be direct treatment or pre-treatment of the molecules.

The term "cell therapeutic agent" as used herein refers to a drug used for the purpose of treatment, diagnosis, and prevention through a series of actions such as proliferation, selection, or changes in the biological characteristics of cells by other methods with living autologous, allogenic, and xenogenic cells in vitro in order to restore a function of cells and tissues.

The cell therapeutic agent may be administered to the human body through any general route as long as it may reach a target tissue.

The pharmaceutical composition of the present disclosure may be prepared using a pharmaceutically suitable and physiologically acceptable adjuvant in addition to the active ingredient, wherein solubilizers such as an excipient, disintegrant, sweetener, binder, coating agent, antifriction agent, lubricant, gliding agent, or a flavoring agent may be used as the adjuvant. The pharmaceutical composition of the present disclosure may preferably be prepared as a medicinal composition including one or more types of pharmaceutically acceptable carriers in addition to the active ingredient for administration. In a composition prepared in a liquid solution, acceptable pharmaceutical carriers including saline, sterile water, Ringer's solution, buffer saline, albumin injection solution, dextrose solution, maltodextrin solution, glycerol, ethanol and one or more components thereof that are sterile and suitable in the body may be mixed and used, and other conventional additives such as antioxidants, buffers, and bacteriostatic agents may be added as needed. In addition, diluents, dispersants, surfactants, binders, and lubricants may be additionally added to prepare injectable formulations such as aqueous solutions, suspensions, and emulsions, pills, capsules, granules, or tablets.

The pharmaceutical preparation form of the pharmaceutical composition of the present disclosure may be granules, acids, coated tablets, tablets, capsules, suppositories, syrups, juices, suspensions, emulsions, drip agents or injectable liquid, and slow-release preparation of active compounds. A medicinal composition of the present disclosure may be administered in a conventional manner through intravenous, intra-arterial, intraperotoneal, intramuscular, intra-arterial, intraperotoneal, intrasternal, percutaneous, intranasal, inhalation, topical, rectal, oral, intraocular, or intradermal routes. The effective dose of the active ingredient in the medicinal composition of the present disclosure refers to an amount required for preventing or treating a disease. Thus, it may be adjusted by various factors including the type of a disease, the severity of the disease, the type and content of the active ingredient and other components included in the composition, the type of formulation and the patient's age, weight, general health status, gender, and diet, administration time, administration route and secretion rate of the composition, duration of treatment, and drugs used together.

The present disclosure provides a method of isolating stem cells without the use of any exogenous protease and without tissue dissociation in the process of isolating solid tissue-resident stem cells. Since exogenous protease is not used, the tissue may preserve its function and structure after isolating stem cells, thereby providing a method that enables the use as a tissue source for repetitive isolation of stem cells from tissues.

It is known that tissue-resident stem cells account for a very small proportion of the cells that constitute the tissue and are usually less than 0.01%. After tissue damage, the somatic cell count decreases, and stem cells to replace or regenerate these cells are activated, leading to an increase in a frequency of stem cells compared to before damage. After damage, stem cells in tissues self-replicate through cell division to produce daughter progenitor cells, and the daughter stem cells migrate to the damaged site. This patent provides a method of inducing self-replication and cell division of stem cells in tissue fragments by activating the tissue homeostasis mechanism in vivo through in vitro culture and recruiting self-replicated and divided stem cells in vitro to the damaged site.

After tissue damage, provisional matrix is formed around the damaged tissue, through which the matrix-cellular integrin pathway is activated, resulting in initiation of a tissue regeneration mechanism as stem cells acting on tissue damage migrate to the damaged site after cell division. The present disclosure provides a method of activating stem cells in tissues, inducing cell division, growth, and migration to a provisional matrix-mimicking hydrogel, and isolating stem cells migrated into the hydrogel, by maximizing matrix-cellular integrin interactions through a 3D support of the provisional matrix-mimicking hydrogel around tissue fragments.

It has been reported that, in response to tissue damage, tissue-resident stem cells are activated, and activated stem cells have increased uPAR or nestin expression. uPAR and nestin expression have been revealed to play an important role in stem cell growth and migration, and uPAR+ and nestin+ cells are known to play a pivotal role in tissue regeneration. Since uPAR and nestin have increased expression in reparative stem cells, uPAR and nestin may be applied as key target markers in stem cell isolation. The present disclosure provides a method of selectively isolating and collecting uPAR+ stem cells by inducing cell division, growth, and migration of tissue-resident uPAR+ and nestin+ stem cells as a result of activation of a matrix-cellular integrin pathway through in vitro culture.

In addition, the present disclosure provides a method of isolating and collecting cells from a hydrogel without exogenous protease according to uPAR expression and plasmin activity of stem cells migrated into and grown in the hydrogel after inducing migration into and growth in the provisional matrix-mimicking hydrogel provided upon 3D culture according to the uPAR-plasmin-MMP activity as a result of physiological responses to activation and stimulation of stem cells.

The present disclosure provides a common method of isolating tissue-resident uPAR+ and nestin+ stem cells from representative solid tissues such as fat, bone marrow, myocardium, nerves, skeletal muscle, and synovium. Tissue-resident uPAR+ and nestin+ stem cells have an ability in self-replication, high in vitro growth, multipotency, and high tissue regeneration, such that the stem cells may be used as regenerative therapeutic agents.

### Modes for Carrying Out the Invention

Hereinafter, the present disclosure will be described in more detail through example embodiments. These example embodiments are only for the purpose of describing the present disclosure in more detail, and it will be apparent to those skilled in the art that the scope of the present disclosure is not limited by these example embodiments according to the gist of the present disclosure.

### <Example 1> Preparation of provisional matrix-mimicking hydrogel

Provisional matrix-mimicking hydrogels were prepared by mixing fibrinogen, collagen, gelatin, or these polymers. Plasma-derived fibrinogen was dissolved in phosphate-buffered saline (PBS) including 10 to 50 mM CaCl₂ at a concentration of 2.5 to 10 mg/ml to prepare a fibrinogen solution. Thrombin was dissolved in PBS at a concentration of 1 to 10 units/ml to prepare a thrombin solution.

Dermis-derived collagen (Matrix BioScience, Germany) or gelatin (befMatrix Collagen, Nitta Gelatin, Japan) was dissolved in 0.1 % (wt./vol.) acetic acid to use a collagen solution at a concentration of 1.0 ~ 20.0 mg/ml. 10X reconstitution buffer for preparing a neutral gelatin or collagen solution was prepared with 50 mM NaHCO₃, 40 mM HEPES, and 0.01 N NaOH and mixed with collagen or gelatin solution at a ratio of 9:1 to prepare a neutral collagen or gelatin solution.

The provisional matrix-mimicking hydrogel was prepared by producing fibrinogen hydrogel using a fibrinogen solution at a concentration of 0.25 to 2.5% and a thrombin solution at a concentration of 0.5 to 5 I.U./mL and mixing a collagen or gelatin solution in a concentration range of 0.1 to 0.5% during the preparation of the fibrin hydrogel to prepare a fibrin/collagen or fibrin/gelatin mixed hydrogel.

### <Example 2> 2-dimensional (2D) and 3-dimensional (3D) organ culture

Organs used for culture include an adipose tissue (AT), bone marrow (BM), myocardium (Myocar), peripheral nerve (PN), skeletal muscle (SM), and synovium (Syn). The study using tissues derived from brain-dead donors was approved by the Institutional Ethics Review Committee, and tissues were donated from brain-dead patients. After removing the hematoma and fibrous tissue attached to the surrounding tissues with scissors, the donated tissue was finely cut into small sections of 0.2 to 2 mm³ in size using a surgical scalpel. Thereafter, the tissue fragments were suspended with PBS, centrifuged at 1,000 rpm, and the washing process was repeated three times to remove a supernatant.

After washing, the tissue fragments were suspended in a culture medium and then cultured by two organ culture methods. In the first method, defined as a monolayer (2D) organ culture method was a case of seeding tissue fragments suspended in the culture medium into a culture vessel and culturing on the surface of the culture vessel.

In the second method, defined as a 3D organ culture method was a method of embedding tissue fragments in fibrin, fibrin/collagen, or fibrin/gelatin provisional matrix-mimicking hydrogel and culturing in a 3-dimensional (3D) environment. In the 3D organ culture method, tissue fragments were mixed with 0.25 ~ 2.5% fibrinogen solution and then with the same amount of 0.5 ~ 5 unit/mL thrombin solution at a 1:1 dose, and the mixture was subjected to polymerization at 37°C for 1 hour to embed the tissue fragments into the fibrin hydrogel. For fat, bone marrow, heart, and muscle, tissue fragments were embedded in the provisional matrix-mimicking fibrin hydrogel with a final 0.25 ~ 1.25% fibrinogen solution and 0.25 ~ 2.5 unit/mL thrombin solution. For nerve and synovial tissue fragments, the tissue fragments were embedded in the provisional matrix-mimicking fibrin/collagen or gelatin hydrogel consisting of 0.25 ~ 2.5% fibrinogen, 0.1 ~ 0.5% neutral collagen or gelatin solution, and 0.25 ~ 2.5 unit/mL thrombin solution.

The tissue fragments mixed with provisional matrix-mimicking hydrogel solution were transferred to a 100-mm to 150-mm culture vessel, which was then placed in an incubator at 37°C for 1 hour and converted to gel after a polymerization process. A composition of the organ culture medium includes 45% v/v DMEM, 45% v/v Ham's F12, 10% fetal bovine serum (FBS, Invitrogen), 20 ng/ml EGF, 2 ng/ml bFGF, 10 ng/ml IGF, and 10 µg/ml gentamicin (Invitrogen). The culture medium was added in an amount equal to twice the amount of gel, and after adding the culture medium, the culture vessel was placed on an orbital shaker for culture for 14 days with stirring at a rate of 30 rpm. The culture medium was replaced twice a week.

In order to inhibit hydrogel degradation, PAI such as tranexamic acid or aminomethyl benzoic acid was added to the culture medium at a concentration of 10 ~ 500 µg/mL daily during a culture period. For the culture of fat, bone marrow, myocardial, and skeletal muscle organs, tranexamic acid or aminomethyl benzoic acid was added at a concentration of 100 ~ 250 µg/mL to the culture medium to inhibit degradation of the provisional matrix-mimicking hydrogel. In the case of peripheral nerves and synovium, tranexamic acid or aminomethyl benzoic acid was added to the culture medium at a concentration of 250 ~ 500 µg/mL, followed by culture.

### <Example 3> Expression of pFAK, uPAR, nestin, and Ki-67 in tissue fragments according to organ culture

After 7 days of 2D and 3D organ culture, cultured adipose tissue, bone marrow, myocardium, nerves, skeletal muscle, and synovial tissue fragments were collected from the culture vessel or hydrogel. A paraffin block was prepared after 2 hours of fixation using a 4% neutral formalin solution. Sections in a thickness of 4 µm were peeled from the paraffin block, and immunohistochemistry staining was performed.

In order to evaluate a degree of activity of integrin-FAK cell signaling pathways in cultured tissue, primary antibodies such as anti-phospho FAK (3283, Cell Signaling Technology, USA; pFAK), anti-uPAR (MAB807, R&D Systems, USA), and Ki-67 (M7240, DAKO, USA) were used to evaluate expression. After a reaction with the primary antibody, the expression rate and positive cell location were evaluated after undergoing three times of washing, a reaction with HRP-conjugated secondary antibody (ImmPRESS One-Step Polymer Systems, Vector Laboratories, USA), color development using DAB as a substrate, and then counter staining with hematoxylin (H-3401-500, Vector Laboratories).

Depending on the in vitro organ culture, the constituent cells in the tissue fragments were activated, and the activated cells proliferated after cell division to operate tissue homeostasis mechanisms to replace or regenerate damaged or lost cells. The integrin cell signaling pathway is one of the main mechanisms to induce tissue regeneration, and integrin cell signaling is activated by the binding of the cell ligand, the extracellular matrix, and receptors. Analyzed in this example embodiment was expression of pFAK, uPAR, and nestin proteins which are target factors activated as a result of integrin cell signaling in constituent cells inside the tissue fragments cultured under monolayer culture (2-dimension, 2D) in which the tissue fragments were cultured on the surface of a culture vessel after seeded directly in the culture vessel and 3-dimensional (3D) environment after embedding in the hydrogel.

After 3D organ culture, it was found that the pFAK expression rate in tissue fragments was significantly higher than that of 2D organ culture. Differences in pFAK expression rate after organ culture depending on the tissue type were identified, and pFAK expression was significantly higher after 3D organ culture in myocardium, nerve, and skeletal muscle (FIG. 1A).

Expression of uPAR, a downstream factor of the integrin signaling pathway, was also significantly high under the 3D organ culture environment with a tendency similar to pFAK, and high expression was observed in myocardium, nerves, and skeletal muscle (FIG. 1B).

Through the integrin signaling, it was possible to induce cell division and growth of constituent cells in organs. Cell regeneration and cell growth ability were evaluated through expression of nestin and Ki-67 which are specifically expressed in cells undergoing regeneration or cell division. The expression rate of nestin in the constituent cells in the tissue fragments after 3D organ culture was significantly high compared to expression of nestin in tissues after 2D organ culture (FIG. 1C). The expression rate of Ki-67, a marker of cell division, was also significantly high in tissue fragments after 3D organ culture (FIG. 1D).

It was possible to induce cell division and growth of cells constituting tissue fragments through in vitro organ culture, maximize integrin signaling with a ligand capable of binding to integrin through a 3D culture environment, and enhance expression of pFAK and uPAR which are downstream target factors, and thus it was found that 3D organ culture induced significantly high cell division and cell growth of the constituent cells in the tissue fragments compared to 2D organ culture.

3D physical stimulation of provisional matrix-mimicking hydrogels may provide a method of significantly increasing division and growth of cells by activating signaling pathways of constituent cells in organs.

### <Example 4> Location and characteristics ofpFAK+, uPAR+, and nestin+ cells in tissue fragments after 3D organ culture

Myocardial tissue fragments cultured before 3D organ culture (0 d) or 3, 5, 7 and 14 days after culture were collected from hydrogel. A paraffin block was prepared after 2 hours of fixation using a 4% neutral formalin solution. Sections in a thickness of 4 µm were obtained from the paraffin block, and immunohistochemistry staining was performed. In order to evaluate a degree of activity of integrin-FAK cell signaling pathways in tissues after culture, primary antibodies such as anti-phospho FAK (3283, Cell Signaling Technology, USA; pFAK), anti-uPAR (MAB807, R&D Systems, USA), nestin (MAB5326, Millipore, USA), and Ki-67 (M7240, DAKO, USA) were used to evaluate expression. After a reaction with the primary antibody, the expression rate and location of positive cells were evaluated after undergoing three times of washing, a reaction with HRP-conjugated secondary antibody (ImmPRESS One-Step Polymer Systems, Vector Laboratories, USA), color development using DAB as a substrate, and counter staining with hematoxylin (H-3401-500, Vector Laboratories).

After encapsulating myocardium in the provisional matrix-mimicking hydrogel, 3D organ culture was performed, and the frequencies of pFAK+, uPAR+, and nestin+ cells in the myocardium were identified. Before culture, expression rates of pFAK, uPAR, and nestin were less than 6%. However, pFAK, uPAR, and nestin-positive cell counts increased significantly in proportion to a culture period after 3D organ culture. After 2 weeks of culture, expression rates of pFAK, uPAR, and nestin in tissue fragments were greater than or equal to 30% (FIGS. 2 and 3). pFAK, uPAR, and nestin were expressed in cells in the stroma between mature somatic cells, and pFAK, uPAR, and nestin were expressed in pericapillary pericytes rather than capillary endothelial cells.

Ki-67, a marker for cell division, was not expressed in mature somatic cells, but expressed by less than 1% before culture. However, the Ki-67 expression rate after in vitro 3D organ culture increased in proportion to the culture period, and the Ki-67 expression rate in the cells residing in the myocardium after 2 weeks of organ culture was 45.7%, with a result that division and growth of cells within the myocardium were induced through 3D organ culture while Ki-67 was also mainly expressed in perivascular cells (FIG. 4).

In this example embodiment, it was found that cell division and growth in myocardium were inducible through the 3D provisional matrix-mimicking hydrogel, and division and growth of pFAK+, uPAR+, and nestin+ vascular pericytes were inducible through organ culture.

### <Example 5> Induction of migration into and growth in provisional matrix-mimicking hydrogel of uPAR+ and nestin+ cells in tissues through 3D organ culture

Fat, bone marrow, myocardium, nerve, skeletal muscle, and synovial tissue fragments were encapsulated in fibrin or fibrin/collagen hydrogel, and 3D organ culture was performed. The organ culture medium was added twice an amount of hydrogel, and the culture vessel was placed on an orbital shaker and subjected to culture for 14 days with stirring at a rate of 30 rpm. PAI was added to the culture medium to inhibit hydrogel degradation. 5 µM bromodeoxyuridine (B5002, Sigma-Aldrich, USA) was added to the culture medium daily for 7 days to label cells that had undergone cell division during in vitro culture. The culture was performed for 2 weeks with culture medium replacement involved twice a week.

After 3, 7 and 14 days of 3D organ culture, the cultured tissue and hydrogel were collected at the same time. A paraffin block was prepared after 2 hours of fixation using a 4% neutral formalin solution. Sections in a thickness of 4 µm were peeled from the paraffin block, and immunohistochemistry staining was performed.

To evaluate expression of uPAR, nestin, and BrdU in cells migrated into and grown in the hydrogel, anti-uPAR (MAB807, R&D Systems, USA), -nestin (MAB5326, Millipore, USA), and -BrdU (347580, BD Biosciences, USA) primary antibodies were used. After a reaction with the primary antibody, expression rate was evaluated by undergoing three times of washing, a reaction with HRP-conjugated secondary antibody (ImmPRESS One-Step Polymer Systems, Vector Laboratories, USA), color development using DAB as a substrate, and counter staining with hematoxylin (H-3401-500, Vector Laboratories).

The provisional matrix-mimicking hydrogel plays a role as an extracellular matrix capable of supporting cell migration and growth, which is achievable by provision of fibronectin, collagen, and fibrin, which are cell adhesion factors essential for cell migration. By activating integrin-pFAK cell signaling pathway through receptor-ligand binding via 3D provisional matrix hydrogel, it was possible to induce cell division and growth of uPAR+ and nestin+ cells in the organ, and induce recruitment, migration, and growth of uPAR+ and nestin+ cells into provisional matrix-mimicking hydrogel.

It was observed that the number of cells that were migrating into and growing in the hydrogel increased in proportion to the 3D myocardial organ culture period (FIG. 5). It was possible to observe that the cells migrated into and grown in the hydrogel combined with the hydrogel in a spindle shape to cause expansion of cytoplasm.

Cells migrated into and grown in a hydrogel from myocardium had uPAR and nestin expression rates of 88.5% and 95.4% or more, showing the same uPAR+ and nestin+ characteristics as the cells that had undergone division and growth after culturing the organ in the tissue. More than 89.4% of the cells migrated in the hydrogel were BrdU positive, which were cells that synthesized DNA by uptaking BrdU that is added during organ culture, such that the majority of the cells in the hydrogel were cells that migrated after the cells were divided and grown after organ culture. BrdU+ cells were not detected in myocardium before organ culture, and uPAR+ and nestin+ cells were less than 2%.

After 2 weeks of 3D organ culture of fat, bone marrow, myocardium, nerve, skeletal muscle, and synovial tissue fragments, the cells in the provisional matrix-mimicking hydrogel were around 90% uPAR positive and more than 90% BrdU positive, and through organ culture, it was found that the tissue-resident cells were cells that divided, grew, and migrated to the hydrogel, with no significant difference observed according to the tissue (FIG. 6).

### <Example 6> Isolation of cells migrated into and grown in a hydrogel after 3D organ culture

Fat, bone marrow, myocardium, nerve, skeletal muscle, and synovial tissue fragments were embedded in fibrin or fibrin/collagen hydrogel, and 3D organ culture was performed. The organ culture medium was added twice an amount of hydrogel, the culture vessel was placed on an orbital shaker, and culture was performed for 14 days with stirring at a rate of 30 rpm. In order to inhibit hydrogel degradation, PAI was added daily during the culture period to inhibit degradation of provisional matrix-mimicking hydrogel according to the PA activity of cells migrated into and grown in a hydrogel.

The uPAR mRNA and plasmin activity of tissue fragments collected after 3D organ culture were evaluated. After preparing tissue lysate from 10 mg of tissue fragments, plasmin activity was analyzed using a Plasmin Activity Assay Kit (ab204728, abcam). After adding fluorescent substrate to tissue lysate, plasmin activity was measured by measuring fluorescence intensity after a reaction at 37°C for 20 minutes. Plasmin activity was calculated by the following formula, ΔRFU360/450nm = (RFU₂-RFU_{2BG})-(RFU₁-RFU_{1BG}). The uPAR mRNA expression rate in tissue fragments was analyzed via real-time qPCR by extracting total RNA from 10 mg of tissue fragments and performing a reverse transcriptase reaction to construct cDNA.

After 14 days of 3D organ culture, the culture medium was removed. After adding DMEM, washing was performed with shaking for 30 minutes at a rate of 30 rpm on an orbital shaker, and the washing solution was removed. This washing process was repeated three times. After washing, the cells migrated into and grown in the hydrogel were isolated and collected by the following two ways. In the first method, 1,000 unit/mL urokinase was added to the culture medium which was then added to a culture vessel, the hydrogel was degraded for 2 hours, the cells and tissue fragments released from the degraded hydrogel were transferred to a tube, the supernatant was removed after centrifugation for 10 minutes at 3,000 rpm, and the collected cell and tissue fragments pellets were suspended in culture medium.

It is possible to isolate and collect cells from hydrogel by PAI withdrawal method. After organ culture for 14 days, the culture medium was removed, and then washing was performed three times using DMEM to remove the PAI remaining in the culture medium and hydrogel. After adding fresh culture medium, culture was performed for 1 hour with shaking at a rate of 30 rpm on an orbital shaker, and PAI in the culture medium was not added. The cells isolated and released from the hydrogel were collected using a transfer pipette, transferred to tubes, and centrifuged at 3,000 rpm for 10 minutes, the supernatant was removed, and the collected cell and tissue fragments pellets were suspended with a culture medium.

The cells isolated and collected from the hydrogel were suspended in the culture medium, the total cell count was calculated by the hemocytometer, and the uPAR expression rate of the collected cells was analyzed using flow cytometry.

Provided in this example embodiment is a method of selectively isolating and collecting uPAR+/nestin+ cells by inducing migration and growth in the hydrogel of uPAR+/nestin+ cells grown after cell division in tissues after 3D organ culture and, regarding cells migrated into and grown in the hydrogel, degrading the hydrogel according to the plasmin activity of the uPAR+/nestin+ cell itself without exogenous protease.

uPAR mRNA expression and plasmin activity in tissue fragments before and after organ culture were evaluated. Even before in vitro culture, uPAR mRNA expression and plasmin activity varied depending on the organ, and uPAR mRNA expression and plasmin activity were high in myocardium, nerves, and skeletal muscle compared to other tissues (FIG. 7). In all tissue fragments evaluated after organ culture, uPAR mRNA expression and plasmin activity were significantly increased compared to that before culture. In particular, mRNA expression and plasmin activity were measured highest in myocardium, neurons, and skeletal muscles. Since plasmin activity was proportional to uPAR expression, it was possible to induce an increase in uPAR expression in tissues through organ culture, and a method of increasing a plasma activity in tissues through uPAR increase after organ culture is provided.

Plasminogen activator inhibitor (PAI) was added in the culture medium during 3D organ culture, and the added PAI may inhibit hydrogel degradation by controlling excessive plasmin and protease activity of organs and cells. When cells subjected to cell division and growth within tissues through organ culture were induced with migration into and growth in a hydrogel and then PAI was withdrawn from the culture medium and hydrogel through rinsing and washing process, it is possible to induce degradation of hydrogel only with organ and cell-resident plasmin and protease activity, and thus cells migrated into and grown in the hydrogel are released, thereby enabling isolation and collection of the released cells.

For PAI withdrawal, the culture medium containing PAI was removed, PAI remaining in the hydrogel and organs was removed through washing three times with DMEM, and the culture medium that did not include PAI was cultured by adding twice the amount of hydrogel. After 30 minutes of culture, the hydrogel began to degrade as a result of PAI withdrawal; with the hydrogel degradation, the cells migrated into and grown in the hydrogel were released from the hydrogel and aggregated between cells; and after 2 hours of culture, hydrogel around tissue fragments and migrated and grown cells were completely degraded, resulting in release of cells in the hydrogel into culture medium (FIG. 8). Through this example embodiment, provisional matrix-mimicking hydrogel was degraded due to cell-resident plasmin and protease activity only with PAI withdrawal, cells in the hydrogel were released into the culture medium, and as a result, it was possible to isolate and collect the released cells in the culture medium in a form having cytoplasm shrunk and aggregated.

It was possible to prevent hydrogel degradation through PAI addition during organ culture, and cells in the hydrogel showed a spindle shape as a result of cytoplasmic spreading. However, through PAI withdrawal in the culture medium, the hydrogel around the cell began to degrade to make the cytoplasm shrunk, cells as a result of cell-cell junction induced the cells to form an aggregate, and the aggregated cells were released into the culture medium (FIG. 9).

In the histological method, it was clearly showed that, though the cells in the hydrogel before PAI withdrawal showed that the cytoplasm was fully spread, when the hydrogel begins to degrade after PAI withdrawal, the hydrogel to which the cells are able to attach was lost, and the cells as a result of cell-cell junction caused the cells aggregated, separated, and released from the hydrogel (FIG. 10). All aggregated cells released from the hydrogel showed characteristics of expressing uPAR, and thus the higher the uPAR expression, the higher the plasmin activity, thereby presenting the basis for isolating uPAR+ cells in the hydrogel only with PAI withdrawal (FIG. 10).

It is possible to isolate and collect cells migrated into and grown in the hydrogel after PAI withdrawal or urokinase treatment. In the present disclosure, comparison and evaluation were conducted on the usefulness of a method of isolating cells migrated into and grown in the hydrogel even with PAI withdrawal without urokinase treatment.

Through an example embodiment, it was possible to identify a result of isolating and collecting cells equivalent to the cell count through urokinase treatment by the PAI withdrawal method (FIG. 11A). More than 2 million cells per 100 mg of tissue could be isolated and collected after organ culture for 2 weeks, which was lower than the number of cells collected after urokinase treatment, but there was no significant difference.

However, the uPAR expression rate of cells collected after PAI withdrawal was significantly higher than that of cells collected after urokinase treatment (FIG. 11B). In particular, the yield of selectively isolating uPAR+ cells from tissues with high uPAR expression rate and plasma activity was high. This showed that the hydrogel degradation activity was high in uPAR+ cells, and as a result, it was possible to verify that it was a highly efficient method of selectively isolating uPAR+ cells by PAI removal and washing.

### <Example 7> Repeated organ culture and cell isolation of tissue fragments collected after 3D organ culture

Fat, bone marrow, myocardium, nerve, skeletal muscle, and synovial tissue fragments were encapsulated in fibrin or fibrin/collagen hydrogel, and 3D organ culture was performed. The organ culture medium was added twice the amount of hydrogel, the culture vessel was placed on an orbital shaker, and culture was performed for 14 days with stirring at a rate of 30 rpm. In order to inhibit degradation of hydrogel, PAI was added daily during the culture period to inhibit degradation of provisional matrix-mimicking hydrogel due to the plasmin activity of cells migrated into and grown in the hydrogel.

After 14 days of 3D organ culture, the culture medium was removed. After adding DMEM, washing was performed with stirring for 30 minutes at a rate of 30 rpm, and the washing solution was removed. The washing process was repeated three times to remove the PAI remaining in the culture medium and hydrogel. After adding fresh culture media, culture was performed for 2 hours with shaking at a rate of 30 rpm, and PAI in the culture medium was not added. The cells and tissue fragments isolated and released from the hydrogel were collected using a transfer pipette, transferred to tubes, and centrifuged at 3,000 rpm for 10 minutes, followed by removal of the supernatant. Since tissue fragments precipitate faster than cells due to high specific gravity, cell and tissue fragments pellets were dispersed in DMEM and then mounted for 30 seconds to induce precipitation of tissue fragments, and then only the supernatant was transferred to a new tube, which was repeated three times to isolate the collected cells and tissue fragments.

The tissue fragments isolated from the hydrogel by the PAI withdrawal method were again encapsulating in the hydrogel, 3D organ culture was performed by the method described in Example 2, and two weeks later, cells and tissue fragments migrated into and grown in the hydrogel by the method described in Example 7 were isolated and collected. The tissue fragments collected after PAI withdrawal were repeatedly induced to cell migration and growth in the tissue through three more consecutive 3D organ cultures so as to isolate and collect cells in the hydrogel, and the total count of isolated and collected cells was measured using a hemocytometer.

In this example embodiment, provided is a method of isolating and collecting cells grown after migrating into the hydrogel after the organ culture by collecting tissue fragments in which a structure is preserved after PAI withdrawal and performing repeated organ culture using the collected tissue fragments. Exogenous protease treatment such as urokinase may degrade the embedded tissue fragments with the hydrogel, resulting in the loss of the structural, functional microenvironment of the tissue fragments.

In this example embodiment, cells and tissue fragments migrated after growth in the tissue without exogenous protease treatment through PAI withdrawal were isolated and collected, and at the same time, the collected tissue fragments were also subjected to repeated organ culture. Three consecutive organ cultures were performed using tissue fragments collected after organ culture, and a result was obtained that cells were collectible at a yield similar to that of the initial organ culture (FIG. 12). It may be determined that the PAI withdrawal method was a method that enables preservation of the structural, functional microenvironment of the tissue fragments embedded with the cells migrated into and grown in the hydrogel after 3D organ culture, and a method of isolating tissue-resident cells with high efficiency and stability through repeated organ culture.

### <Example 8> Culture of cells isolated from hydrogel after PAI withdrawal and urokinase treatment

Fat, bone marrow, myocardium, nerve, skeletal muscle, and synovial tissue fragments were embedded in the fibrin or fibrin/collagen hydrogel, and 3D organ culture was performed. The organ culture medium was added twice the amount of hydrogel, the culture vessel was placed on an orbital shaker, and culture was performed for 14 days with stirring at a rate of 30 rpm. In order to inhibit hydrogel degradation, tranexamic acid, which is PAI, was added to the culture medium daily.

After 14 days of 3D organ culture, the culture medium was removed. After adding DMEM, the medium was washed for 30 minutes while stirring at a rate of 30 rpm, and the washing process was repeated three times. After washing, the cells migrated into and grown in the hydrogel were collected after isolating cells by the PAI withdrawal or urokinase method presented in Example 6. After organ culture for 14 days, the culture medium was removed, and washing was performed three times using DMEM to remove the PAI remaining in the culture medium and hydrogel. After adding fresh culture medium, culture was followed for 1 hour with stirring at a rate of 30 rpm, and PAI in the culture medium was not added. The cells isolated and released from the hydrogel were collected using a transfer pipette, transferred to tubes, and centrifuged at 3,000 rpm for 10 minutes, and then the supernatant was removed, followed by suspension of the collected cell and tissue fragments pellets with a culture medium.

For urokinase treatment, after three times of washing, 1,000 unit/mL urokinase was added to the culture medium, the hydrogel was degraded for 2 hours, and then the cells and tissue fragments released from the degraded hydrogel were collected, transferred to a tube, and centrifuged at 3,000 rpm for 10 minutes to remove the supernatant, followed by suspension of the collected cell and tissue fragments pellets with a culture medium.

The cells isolated and collected from the hydrogel were seeded by 5,000 cells per cm2 of the polystyrene culture vessel, and then the culture medium was added. The adhesion and growth characteristics of cells isolated and collected from hydrogel under a monolayer culture environment were evaluated using a microscope.

After PAI withdrawal, it was observed that cell aggregates were formed as the cells are released from the hydrogel (FIG. 13A); the collected cells were suspended in a culture medium to be formed in small circular aggregates (FIG. 13B); and seeding was performed in a PS culture vessel to culture in a monolayer environment. The cells collected in an intercellular aggregation state adhered to the culture vessel 30 minutes after seeding (FIG. 13C); the cytoplasm of the seeded cells expanded 1 hour after seeding (FIG. 13D); and 2 hours after culture, it was found that the cells stably adhered while cells around the cell aggregate grew (FIG. 13E).

After organ culture of all tissue fragments such as fat, bone marrow, myocardium, nerve, skeletal muscle, and synovium, it was possible to safely isolate and collect cells through PAI withdrawal, and, when the collected cells were mixed with the culture medium and seeded in the culture vessel, it was found that all the collected cells adhered to the culture vessel within 30 minutes while cytoplasm stably expanded, with no significant difference according to tissues (FIG. 14).

Cells seeded and cultured in the monolayer environment showed a high growth rate, grew around seeded cells after 2 days of culture, and expanded to the periphery, and cells isolated and collected through PAI withdrawal were found to maintain a high growth rate even when cultured in a monolayer environment (FIG. 15).

### <Example 9> Immunophenotypic features of cells isolated after PAI withdrawal

Fat, bone marrow, myocardium, nerve, skeletal muscle, and synovial tissue fragments were embedded in the fibrin or fibrin/collagen hydrogel, and 3D organ culture was performed. The organ culture medium was added twice the amount of hydrogel, the culture vessel was placed on an orbital shaker, and culture was performed for 14 days with stirring at a rate of 30 rpm. In order to inhibit hydrogel degradation, tranexamic acid which is PAI was cultured while being added to the culture medium at a concentration.

After 14 days of 3D organ culture, the culture medium was removed. After adding DMEM, washing was performed for 30 minutes with stirring at a rate of 30 rpm, and the washing process was repeated three times. After washing, the cells migrated and grown in the hydrogel were collected after isolating the cells by the PAI withdrawal method. After organ culture for 14 days, the culture medium was removed, and washing was performed three times using DMEM to remove the PAI remaining in the culture medium and hydrogel. After adding fresh culture medium, culture was performed for 1 hour with stirring at a rate of 30 rpm, and PAI in the culture medium was not added. After cells isolated and released from the hydrogel were collected using a transfer pipette, transferred to tubes, and centrifuged at 3,000 rpm for 10 minutes, the supernatant was removed, and the collected cell and tissue fragments pellets were suspended in a culture medium.

Cells isolated and collected after PAI withdrawal method or urokinase treatment were suspended in PBS, and then immunophenotypic features were analyzed using flow cytometry. Expression rates of mesenchymal stem cell (MSC) markers CD29, CD44, CD105, and CD140b, as well as uPAR and nestin were evaluated. CD34 and CD45 as hematopoietic cell markers and CD31 as a vascular endothelial cell marker were used to evaluate the expression rate of these markers.

As a result of urokinase treatment or PAI withdrawal, it was possible to collect and isolate cells that have migrated and grown from hydrogel. Regardless of the method of collecting the cells, the cells that migrated and grew in the hydrogel from all cultured tissues showed the same immunophenotypic features as mesenchymal stem cells with expression rates for CD29, CD73, CD105, and CD140b greater than 90% (FIG. 16A). There was no difference in the expression rate of mesenchymal stem cell markers in cells isolated and collected from the hydrogel according to the isolation method.

However, in this Example, expression rates of uPAR and nestin were more than 90% in cells isolated and collected by PAI withdrawal after organ culture of all tissue fragments. On the other hand, the expression rate of cells collected after urokinase treatment was in the range of 30 ~ 67%. Depending on the high plasmin activity of uPAR+ and nestin+ cells in the hydrogel, it was able to derive a result that selective isolation and collection was possible through PAI withdrawal. On the other hand, it was determined in the urokinase treatment that cells with various immunophenotypic features in the hydrogel were mixed and collected (FIG. 16B).

Hematopoietic cells and vascular endothelial cells present in tissues may be mixed during the process of isolating cells from tissues, and the degree of mixing of these cells may be identified by detecting CD31+, CD34+, and CD45+ cells. In the case of cells isolated and collected from the hydrogel by PAI withdrawal method, the expression rates of CD31, CD34, and CD45 were less than 1%, but the cells isolated and collected after urokinase treatment were 2.8 ~ 7.0%, thereby finding that the mixing degree of hematopoietic cells and vascular endothelial cells was high (FIG. 17).

Through this example embodiment, it was found that it was a method of selectively isolating and collecting uPAR+ and nestin+ cells that have migrated from the tissue into the hydrogel through PAI withdrawal, while the mixing of hematopoietic cells and vascular endothelial cells during the isolation process was minimized.

### <Example 10> Self-replication ability of uPAR+/nestin+ cells collected and isolated after PAI withdrawal

Fat, bone marrow, myocardium, nerve, skeletal muscle, and synovial tissue fragments were embedded in the fibrin or fibrin/collagen hydrogel, 3D organ culture was performed for 2 weeks, and then the cells migrated into and grown in the hydrogel were isolated and collected by the PAI withdrawal and urokinase method described in Example 6.

The cells isolated and collected from the hydrogel were transferred to tubes and centrifuged for 10 minutes at 3,000 rpm to remove the supernatant. The cell precipitate was suspended in the culture medium, and a cell density was adjusted to 1.0E+06 per mL. In order to verify the self-replication ability of cells isolated and collected from the hydrogel, it was evaluated via a colony forming assay (CFU). After 6,000 cells were seeded in a 100-mm culture vessel, culture was performed for 2 weeks, followed by staining with 1% crystal violet after culture. After washing, the number of colonies with a diameter of 2 mm or more was measured.

The frequency of CFU formation according to tissue type differed, but was significantly high in cells isolated and collected from the hydrogel through PAI withdrawal in all tissue fragments compared to cells collected after urokinase treatment (FIG. 18). In particular, cells derived from myocardium, nerves, and skeletal muscle showed a high colony forming ability compared to cells derived from other tissues. In the case of cells isolated after urokinase treatment, the CFU frequency was 3.1 ~ 8.4%, but the cells isolated by the PAI withdrawal method were found to have a high CFU frequency of 8.8 ~ 37.9%.

Self-replication ability is one of the main characteristics of stem cells, 3D organ culture may activate stem cells in tissues to induce migration into and growth in hydrogel, and stem cells in hydrogel may be selectively isolated and collected with a high self-replication titer through the PAI withdrawal method, proving that uPAR+/nestin+ cells with high plasma activity were stem cells with the high titer (FIG. 18).

### <Example 11> In vitro growth ability of cells isolated after PAI withdrawal

Fat, bone marrow, myocardium, nerve, skeletal muscle, and synovial membrane tissue fragments were embedded in the fibrin or fibrin/collagen hydrogel, 3D organ culture was performed for 2 weeks, and then the cells migrated into and grown in the hydrogel were isolated and collected by the PAI withdrawal and urokinase methods described in Example 6.

The cells isolated and collected from the hydrogel were transferred to tubes and centrifuged for 10 minutes at 3,000 rpm to remove the supernatant. The cell precipitate was suspended in the culture medium, and a cell density was adjusted to 1.0E+06 per mL. In order to verify the in vitro growth ability of cells isolated and collected from hydrogel, population doubling time (PDT) and population doubling level (PDL) were analyzed and evaluated. After seeding 3,000 cells per cm² in T75 flask, culture was performed for 7 days, the cells were collected after trypsin/EDTA treatment, and the total cell count was calculated by a hemocytometer. PDT and PDL were calculated and compared through the number of seeded cells, the total number of cells collected, and the culture period. PDT was calculated according to the following formula. Calculation was conducted by the formula, PDT = [(culture time)/((logN2-logN1)/log2)], wherein N1 is the cell count at the time of seeding, and N2 is the cell count collected after culture. PDL was calculated according to the following formula. Calculation was conducted by the formula, PDL = [PDL0 + 3.322(logN2-logN1)], wherein N1 is the cell count at the time of seeding, and N2 is the cell count collected after culture.

The higher the in vitro growth ability, the higher the expression rate of Ki-67, a cell cycle marker, in cells being cultured. In vitro cell growth ability was subjected to comparative analysis by analyzing the anti-Ki67 expression rate by flow cytometry.

After urokinase or PAI withdrawal treatment, cells migrated and grown from the hydrogel were isolated, and the in vitro growth ability of the collected cells was compared and analyzed. Compared with cells obtained after urokinase treatment, PDT was significantly low and PDL was significantly high in cells isolated and collected through PAI withdrawal. With the same tendency as self-replication ability, it was found that the cells had excellent in vitro growth ability with low PDT and high PDL of cells isolated and collected through PAI withdrawal (FIG. 19).

In order to verify a high growth ability of cells isolated and collected from hydrogel after PAI withdrawal treatment, Ki-67 expression rate was analyzed through flow cytometry. Ki-67 markers refer to cells in the cell cycle, so the higher Ki-67 expression rate refers to an indicator with the higher cell growth ability. Cells obtained through PAI withdrawal showed a high Ki-67 expression rate of 58.5 ~ 75.4%, which was significantly higher than 33.6 ~ 48.7% in cells obtained after urokinase treatment (FIG. 20).

As a result of the above, it may be found that PAI withdrawal was a method of selectively isolating cells with high in vitro growth ability, which supports that uPAR+/nestin+ cells with high plasma activity were cells with high growth ability.

### <Example 12> Differentiation ability of cells isolated after PAI withdrawal

Tissue fragments of fat, bone marrow, myocardium, nerve, skeletal muscle, and synovial were embedded in the fibrin or fibrin/collagen hydrogel, 3D organ culture was performed for 2 weeks, and then cells migrated into and grown in the hydrogel were isolated and collected by the PAI withdrawal and urokinase methods described in Example 6.

The cells isolated and collected from the hydrogel were transferred to tubes and centrifuged for 10 minutes at 3,000 rpm to remove the supernatant. The cell precipitate was suspended in the culture medium, and a density was adjusted to 1.0E+06 per mL. The ability of cells in the hydrogel to differentiate into adipocytes and osteoblasts was evaluated. 2.0E+ 05 cells were seeded in a 24-well culture vessel, and after adding a differentiation medium added with 10% CS, 0.5 mM 3-isobutyl-1-methylxanthine (Sigma), 80 µM indomethacin (Sigma), 1 µM DEX, and 5 µg/mL insulin was added to 90% DMEM, culture was performed for 14 days. The degree of differentiation of stem cells into adipocytes was evaluated using Triglyceride-Glo kit (Promega) by performing staining with 0.5% Oil Red O (Sigma) solution, which is an indicator of fat accumulation in cytoplasm, at room temperature for 1 hour 2 weeks after induction of differentiation and then measuring triglyceride in the cytoplasm. In order to evaluate the ability to differentiate into osteoblasts, 2.0E+ 05 cells were seeded in a 24-well culture vessel, α-MEM including 1 µM DEX, 50 µM ascorbic acid, 10 mM β-glycerol phosphate, and 10% calf serum was added, and culture was performed for 2 weeks to induce differentiation. Comparative analysis was conducted on whether differentiation into osteoblasts occurred via optical density by performing staining after adding Alizarin Red (Sigma) 2 weeks after induction of differentiation and also on the degree of mineral deposition by measuring absorbance at 520 nm.

In FIG. 21, after differentiation of cells isolated and collected from bone marrow into osteoblasts (left) and adipocytes (right) was induced, accumulation and deposition of calcium phosphate crystals could be observed through alizarin red staining, and accumulation of lipids in the cytoplasm of cells induced to differentiate into adipocytes could be observed through oil red O staining. It was found that all cells derived from all tissue fragments had the ability to differentiate into osteoblasts and adipocytes.

The differentiation ability into osteoblasts and adipocytes was compared and evaluated by OD values measured after dissolving deposited or accumulated mineral crystal and fat vacuole. It was found that the ability to differentiate into osteoblasts and adipocytes was significantly high in cells isolated and collected from hydrogel by PAI withdrawal in all tissue fragments compared to that of cells isolated and collected after urokinase treatment (FIG. 22). Differentiation into osteoblasts showed high ability in bone marrow, myocardium, and skeletal muscle-derived cells, and differentiation into adipocytes showed high ability in fat, skeletal muscle, and synovium. However, by identifying the differentiation ability into osteoblasts and adipocytes that were significantly high in cells isolated and collected from hydrogel by the PAI withdrawal method regardless of origin of tissue fragments, it was found that it was a stem cell with high multipotency in uPAR+/nestin+ cells with high plasmin activity.

### <Example 13> Ability to induce tissue regeneration of cells isolated after PAI withdrawal

Tissue fragments of fat, bone marrow, myocardium, nerve, skeletal muscle, and synovial were embedded in the fibrin or fibrin/collagen hydrogel, 3D organ culture was performed for 2 weeks, and then the cells migrated into and grown in the hydrogel were isolated and collected by the PAI withdrawal and urokinase methods described in Example 6.

The cells isolated and collected from the hydrogel were transferred to tubes and centrifuged for 10 minutes at 3,000 rpm to remove the supernatant. The cell precipitate was suspended in the culture medium, and a cell density was adjusted to 1.0E+06 per mL. After seeding 5.0E+ 06 cells in T175 flask, culture was performed for 3 days, 1 mL of TRIzol was added, and then cell lysates were collected and frozen at -20 degrees until total RNA was isolated. Total RNA was extracted and purified using a PureLink RNA kit (Thermo Fisher), and cDNA was synthesized using reverse transcriptase. Expression of basic FGF (bFGF), HGF, IGF, and sdf-1 mRNA, which play a major role in tissue protection and regeneration, was compared and evaluated by calculation using the expression rate compared to cord blood-derived mesenchymal stem cells (CB-MSCs). The target mRNA gene was amplified through real-time gene amplification using a target mRNA-specific initiator and a SYBR green real-time PCR kit, and the mRNA expression rate compared to umbilical cord blood-derived mesenchymal stem cells (CB-MSCs) was evaluated by the 2-ΔΔCt method.

Cells migrated and grown from hydrogel after urokinase or PAI withdrawal treatment were isolated, and the ability to induce tissue regeneration of collected cells was compared and analyzed through relevant mRNA expression. The expression of genes inducing tissue regeneration differed depending on the tissue of origin, the expression of these genes was high in myocardium, nerve, and skeletal muscle-derived cells, and myocardium-derived cells were high in HGF and SDF-1 mRNA while IGF mRNA expression was high in nerve-derived cells [FIG. 23]. In cells isolated and collected by PAI withdrawal, the expression of whole tissue regeneration genes was significantly high compared cells isolated and collected after urokinase treatment. These results showed that uPAR expression was proportionally associated with high tissue regeneration-regulating gene expression.

### <Example 14> Plasmin activity and nestin expression rate in uPAR+ cells

Fat, bone marrow, myocardium, nerve, skeletal muscle, and synovial tissue fragments were embedded in the fibrin or fibrin/collagen hydrogel, 3D organ culture was performed for 2 weeks, and then cells migrated into and grown in the hydrogel isolated and collected by the urokinase method described in Example 6. Subsequently, uPAR+ (UK+uPAR+) and uPAR- (UK+ uPAR-) cells were isolated through FACSAria (BD Bioscience) and cultured in a monolayer environment for 7 days to evaluate the plasmin activity and nestin expression rate. Cells isolated and collected from hydrogel by PAI withdrawal method had a uPAR expression rate of 90% or more, such that secondary uPAR+ cells were not isolated. Plasmin activity was performed according to the method described in Example 6, and nestin expression rate was evaluated using flow cytometry.

Significantly high plasmin activity was observed in uPAR+ cells (FIG. 24, left). UK+uPAR+ cells isolated and cultured after urokinase treatment and cells isolated and cultured after PAI withdrawal showed significantly high plasmin activity compared to UK+uPAR- cells. The plasmin activity of cells varied depending on the tissue of origin, and was high in myocardium, nerve and skeletal muscles, while plasmin activity was determined based on the tissue of origin of the tissue-resident cells.

The above results showed that there was a close correlation between the plasmin activity and nestin expression rate in uPAR+ cells.

### <Example 15> Self-replication and in vitro growth ability of uPAR+ cells

Fat, bone marrow, myocardium, nerve, skeletal muscle, and synovial tissue fragments were encapsulated in the fibrin or fibrin/collagen hydrogel, and then 3D organ culture was performed for 2 weeks to isolate and collect cells migrated into and grown in the hydrogel using the urokinase method described in Example 6. Subsequently, uPAR+ (UK+uPAR+) and uPAR- (UK+uPAR-) cells were isolated through FACSAria (BD Bioscience) and cultured in a monolayer environment for 7 days, and then self-replication and growth ability were evaluated. Cells isolated and collected from hydrogel by PAI withdrawal method had a uPAR expression rate of 90% or more, such that secondary uPAR+ cells were not isolated. Self-replication ability was evaluated according to the method described in Example 10, and growth ability was evaluated using Ki-67 expression rate via flow cytometry.

It was possible to observe a significantly high self-replication ability in uPAR+ cells. It was found that the CFU frequency in UK+uPAR+ cells and PAI withdrawal cells was 12.5 ~ 37.5% and 3.1 ~ 8.8% in UK+uPAR- cells, with significantly high self-replication ability in uPAR+ cells (FIG. 25, left).

It was found that the self-replication ability and in vitro growth ability were closely related, and the Ki-67 expression rate was 40.8 ~ 82.4% in uPAR+ cells, showing significantly high growth ability compared to the Ki-67 expression rate of 28.1 ~ 45.7% in uPAR- cells (FIG. 25, right). Myocardium, nerve, and skeletal muscle-derived uPAR+ cells had higher CFU formation ability and Ki-67 expression rate compared to tissues of other origins.

### <Example 16> Anti-inflammatory ability ofuPAR+ cells

Fat, bone marrow, myocardium, nerve, skeletal muscle, and synovial tissue fragments were embedded in the fibrin or fibrin/collagen hydrogel, 3D organ culture was performed for 2 weeks, and then cells migrated and grown in the hydrogel were isolated and collected by the urokinase method described in Example 6. Subsequently, uPAR+ (UK+uPAR+) and uPAR- (UK+uPAR-) cells were isolated through FACSAria (BD Bioscience) and cultured in a monolayer environment for 7 days to evaluate the anti-inflammatory ability. Cells isolated and collected from hydrogel by PAI withdrawal method had a uPAR expression rate of 90% or more, such that secondary uPAR+ cells were not isolated.

Anti-inflammatory ability was evaluated using conditioned media including factors secreted by cells. To prepare the conditioned medium, UK+uPAR+, UK+uPAR-, and PAI withdrawal cells were cultured for 1 week after adding DMEM/F12 serum-free medium seeded with 40,000 cells per cm² to T175 flask.

RAW 264.7 cells were suspended in 90% RPMI 1640/10% calf serum, and 100,000 cells per cm² were seeded on a 12-multiwell plate and cultured for 1 day. 100 µg/mL LPS was added to the culture medium to sensitize RAW 264.7 cells. During LPS sensitization, the conditioned media was added to the culture medium in a ratio of 1:10 to evaluate the anti-inflammatory ability. For the anti-inflammatory evaluation index, the isolation levels of TNFα and IL-1β secreted by RAW 264.7 cells were measure via ELISA method for comparison and evaluation.

RAW 264.7 cells showed a 5-fold increase in TNFα and IL-1β secretion levels after LPS stimulation. It was able to determine that substances secreted by tissue-resident stem cells isolated and collected from hydrogel had an ability to inhibit the isolation of inflammatory cytokines of RAW 264.7 cells by 30 ~ 70% (FIG. 26). The conditioned media prepared from UK+uPAR+ cells and PAI withdrawal cells showed significantly high TNFα and IL-1β secretion inhibitory ability compared to UK+uPAR- cells. Regardless of the tissue of origin, it was possible to find a high anti-inflammatory effect of uPAR+ cells compared to uPAR- cells.

### <Example 17> Effect of inducing growth of vascular endothelial cells and fibroblasts of uPAR+ cells

Fat, bone marrow, myocardium, nerve, skeletal muscle, and synovial tissue fragments were embedded in the fibrin or fibrin/collagen hydrogel, 3D organ culture was performed for 2 weeks, and cells migrated into and grown in the hydrogel were isolated and collected by the urokinase method described in Example 6. Subsequently, uPAR+ (UK+uPAR+) and uPAR- (UK+uPAR-) cells were isolated through FACSAria (BD Bioscience) and cultured under a monolayer environment for 7 days to evaluate the anti-inflammatory ability. Cells isolated and collected from hydrogel by PAI withdrawal method had a uPAR expression rate of 90% or more, such that secondary uPAR+ cells were not isolated.

Human umbilical vein endothelial cells (HUVECs) were used for vascular endothelial cells, and human dermal fibroblasts (DFs) were used. The growth inducing effect of the corresponding cells in the conditioned media was tested. The conditioned media prepared by the method described in Example 16 were prepared to test the effect. HUVEC cells and DF were suspended in a 99% DMEM/1% calf serum culture, and then 4,000 cells per cm2 were seeded in a 24-multiwell plate. The conditioned medium was added to the culture medium in a ratio of 1:9 and cultured for 3 days, and the cell count after culture was measured using PicoGreen dsDNA quantitation kit (Invitrogen). The cell growth effect was compared and analyzed by calculating the increase ratio (fold) over the number of cells first seeded.

It was determined that conditioned media prepared from UK+uPAR+ and PAI withdrawal cells showed the effect of promoting HUVEC and DF growth, while the effect was significantly higher than 30% compared to the conditioned medium prepared in UK+uPAR- cells, but there was no difference in the effect between UK+ uPAR+ and PAI withdrawal cells (FIG. 27). In particular, the conditioned media obtained from myocardium, nerve and skeletal muscle-derived stem cells showed a high growth-inducing effect compared to other tissue-derived stem cells.

### <Example 18> Vascular endothelial cell and fibroblast protective effect of uPAR+ cells

Fat, bone marrow, myocardium, nerve, skeletal muscle, and synovial tissue fragments were embedded in the fibrin or fibrin/collagen hydrogel, and then 3D organ culture was performed for 2 weeks to isolate and collect cells migrated and grown in hydrogel by the urokinase method described in Example 6 Subsequently, uPAR+ (UK+uPAR+) and uPAR- (UK+uPAR-) cells were isolated through FACSAria (BD Bioscience) and cultured in a monolayer environment for 7 days to evaluate the anti-inflammatory ability. Cells isolated and collected from hydrogel by PAI withdrawal method had a uPAR expression rate of 90% or more, such that secondary uPAR+ cells were not isolated.

The cytoprotective effect was tested using conditioned media prepared from UK+uPAR- cells, UK+uPAR+ cells, and cells isolated and collected after PAI withdrawal, respectively. HUVEC and DF were suspended in a 99% DMEM/1% calf serum culture medium, and 5,000 cells per cm² were seeded in a 24-multiwell plate. 1 hour before induction of H₂O₂-mediated cell damage, the conditioned media were added with the culture medium in a ratio of 1:9. 0.01% H₂O₂ was added to induce cell damage, and after 6 hours of damage induction, the culture medium was removed and washed twice with PBS. The degree of cell damage was evaluated by annexin V expression rate, and the apoptotic cells were analyzed by flow cytometry after reacting with FITC-conjugated annexin V (BD Biosciences) with cells.

Conditioned media prepared from UK+uPAR+ cells and cells collected after PAI withdrawal treatment showed significant protection against H2O2-mediated HUVEC and DF cell death compared with UK+uPAR- cell-derived conditioned media (FIG. 28). Differences in cytoprotective effects were observed according to the tissues of origin, and HUVEC and DF cytoprotective effects were high in myocardium, nerve, and skeletal muscle-derived cells. The annexin V expression rate was 28.7 ~ 45.1% in HUVEC and DF treated with UK+uPAR+ cell conditioned medium, which showed significantly higher cytoprotective effect than 42.1 ~ 71.3% in cells treated with UK+ uPAR- cell conditioned medium, but no significant difference was identified with the conditioned media derived from PAI withdrawal cells. These results determine that uPAR expression was associated with high cytoprotective effect, and there was evidence that PAI withdrawal alone may enable isolation and culture of cells with high cytoprotective effects.

The present disclosure is achieved by engineered provisional matrix supported organ culture. Tissue fragments were embedded in an artificial provisional matrix, and the integrin-FAK cell signaling pathway was activated through the provisional matrix to induce cell division and growth of tissue-resident stem cells. Through the organ culture process, migration and growth of stem cells in the tissues to provisional matrix increases in proportion to uPAR-uPA-plasmin activity. In order to control a result in which inhibition of migration of stem cells is halted as provisional matrix is degraded and lost due to excessive uPAR-uPA-plasmin activity, it is possible to control excessive degradation of provisional matrix through PAI addition. Provided is a technology that enables isolation of uPAR+ stem cells by stopping PAI addition after migration and growth of stem cells into the provisional matrix reach a target level. Following the cessation of PAI addition, uPAR+ stem cells were dissociated from the provisional matrix and released into the culture medium according to uPAR-uPA-plasmin and MMP activation. The present disclosure provides a technique that enables isolation and culture of uPAR+ stem cells without the use of any tissue-degrading protein and stem cell purification process using markers by migration and degradation characteristics of the provisional matrix according to uPAR expression.

The present disclosure may be applied to isolation and culture of uPAR+ stem cells present in solid tissues such as bone marrow, fat, skeletal muscle, heart, peripheral nerves, spinal cord, brain, lungs, liver, joint membrane, umbilical cord, placenta, and periodontium. The present disclosure provides a method of isolating and culturing stem cells through repeated organ culture since structures of the tissue fragments used for organ culture may be preserved intact by isolating stem cells without treatment of any protease.

The present disclosure shows the characteristics that conventional stem cell markers in addition to uPAR among uPAR-positive tissue-resistant stem cells are positively expressed. In the case of bone marrow, fat, muscle, heart, joint membrane, umbilical cord, and placenta, mesenchymal stem cell markers such as CD29, CD44, CD73, CD90, and CD105 were expressed positively, but hematopoietic stem cell or vascular endothelial cell markers showed negative characteristics. The present disclosure shows that markers such as nestin, p75, Sox10, and Sox2 are co-expressed in uPAR-positive stem cells derived from peripheral nerve, spinal cord, and brain tissues.

The uPAR-positive stem cells of the present disclosure show biological characteristics high in ability of secreting physiologically active factors acting on migration, growth factors, anti-inflammatory factors, stem cell recruitment factors, and tissue regeneration.

The uPAR-positive stem cells of the present disclosure have high multipotency to differentiate into tissue constituent cells.

## Claims

1. A method of inducing tissue-resident uPAR+ and nestin+ stem cells to enter the cell cycle, the method comprising:
(1) preparing a provisional matrix-mimicking hydrogel;
(2) encapsulating isolated tissue fragments into the provisional matrix-mimicking hydrogel; and
(3) 3D culturing the provisional matrix-mimicking hydrogel into which the tissue fragments are encapsulated, in a culture medium to which plasminogen activator inhibitor (PAI) is added.

2. The method of claim 1, wherein the provisional matrix-mimicking hydrogel is a fibrin hydrogel in which a fibrinogen solution at a concentration of 0.25 to 2.5% and a thrombin solution at a concentration of 0.5 to 5 I.U./mL are mixed, a fibrin/collagen mixed hydrogel in which a collagen solution at a concentration of 0.1 to 0.5% is mixed in the fibrin hydrogel, or a fibrin/gelatin mixed hydrogel in which a gelatin solution at a concentration of 0.1 to 0.5% is mixed in the fibrin hydrogel.

3. The method of claim 1, wherein the tissue is adipose tissue, bone marrow tissue, myocardial tissue, peripheral nerve tissue, skeletal muscle tissue, or synovial tissue.

4. The method of claim 1, wherein the PAI is tranexamic acid or aminomethyl benzoic acid.

5. The method of claim 1, wherein the method activates integrin-FAK cell signaling of cells in tissues to induce cell division and cell growth of tissue-resident uPAR+ and nestin+ stem cells.

6. The method of claim 1, wherein the method induces cell migration into the provisional matrix-mimicking hydrogel and cell growth of the tissue-resident uPAR+ and nestin+ stem cells.

7. A method of isolating and culturing tissue-resident uPAR+ and nestin+ stem cells, the method comprising:
(1) preparing a provisional matrix-mimicking hydrogel;
(2) encapsulating isolated tissue fragments into the provisional matrix-mimicking hydrogel;
(3) 3D culturing the provisional matrix-mimicking hydrogel into which the tissue fragments are encapsulated, in a culture medium to which PAI is added;
(4) removing the 3D culture medium and removing PAI by washing;
(5) re-culturing the PAI-removed culture with a PAI-free culture medium to degrade the provisional matrix-mimicking hydrogel; and
(6) isolating stem cells released in the re-culture medium.

8. The method of claim 7, wherein the provisional matrix-mimicking hydrogel is a fibrin hydrogel in which a fibrinogen solution at a concentration of 0.25 to 2.5% and a thrombin solution at a concentration of 0.5 to 5 I.U./mL are mixed, a fibrin/collagen mixed hydrogel in which a collagen solution at a concentration of 0.1 to 0.5% is mixed in the fibrin hydrogel, or a fibrin/gelatin mixed hydrogel in which a gelatin solution at a concentration of 0.1 to 0.5% is mixed in the fibrin hydrogel.

9. The method of claim 7, wherein the tissue is adipose tissue, bone marrow tissue, myocardial tissue, peripheral nerve tissue, skeletal muscle tissue, or synovial tissue.

10. The method of claim 7, wherein the PAI is tranexamic acid or aminomethyl benzoic acid.

11. The method of claim 7, wherein step (5) comprises inducing an increase in uPAR+ cells in the tissue and degrading the provisional matrix-mimicking hydrogel through an increase in a plasmin activity.

12. The method of claim 7, wherein the tissue-resident uPAR+ and nestin+ stem cells have enhanced ability in self-replication, in vitro growth, differentiation, or tissue regeneration induction.

13. The method of claim 7, wherein tissue fragments collected from the re-culture medium in step (5) further comprises a process of repeating steps (2) to (5) 1 to 10 times.

14. Tissue-resident uPAR+ and nestin+ stem cells isolated and cultured according to the method of any one of claims 7 to 13, or a culture thereof.

15. A pharmaceutical composition for preventing or treating inflammatory diseases, comprising the tissue-resident uPAR+ and nestin+ stem cells according to claim 14 or a culture thereof as an active ingredient.

16. A pharmaceutical composition for preventing or treating autoimmune diseases, comprising the tissue-resident uPAR+ and nestin+ stem cells according to claim 14 or a culture thereof as an active ingredient.

17. A pharmaceutical composition for healing wounds, comprising the tissue-resident uPAR+ and nestin+ stem cells according to claim 14 or a culture thereof as an active ingredient.

18. A pharmaceutical composition for promoting vascular regeneration, comprising the tissue-resident uPAR+ and nestin+ stem cells according to claim 14 or a culture thereof as an active ingredient.
